# FASCICULE DE BREVET EUROPEEN

(11) **EP 3 286 139 B1**
(45) Date de publication et mention de la délivrance du brevet: **17.03.2021**
(21) Numéro de dépôt: 16744434.8
(22) Date de dépôt: 15.06.2016
(51) Int. Cl.: C01G 49/08, A61K 33/26

(54) **PRÉPARATION APYROGÈNE CONTENANT DES NANOPARTICULES SYNTHÉTISÉES PAR DES BACTÉRIES MAGNÉTOTACTIQUES POUR DES APPLICATIONS MÉDICALES OU COSMÉTIQUES**
APYROGENES PRÄPARAT MIT DURCH MAGNETOTAKTISCHE BAKTERIEN SYNTHETISIERTEN NANOPARTIKELN FÜR MEDIZINISCHE ODER KOSMETISCHE ANWENDUNGEN
APYROGENIC PREPARATION CONTAINING NANOPARTICLES SYNTHESISED BY MAGNETOTACTIC BACTERIA FOR MEDICAL OR COSMETIC APPLICATIONS

(30) Priorité: 17.06.2015 FR 1501267
(43) Date de publication de la demande: 28.02.2018
(73) Titulaire: Nanobacterie, 75116 Paris (FR)
(72) Inventeur: ALPHANDÉRY, Edouard, 75116 Paris (FR); DURAND-DUBIEF, Mickaël, 92380 Garches (FR)
(86) Numéro de dépôt international: PCT/FR2016/000095
(87) Numéro de publication internationale: WO 2016/203121

(56) Documents cités:
- WO-A1-2011/061259
- GB-A- 2 464 998
- Jin Xie ET AL: "Production, Modifi cation and Bio-Applications of Magnetic Nanoparticles Gestated by Magnetotactic Bacteria", , 31 décembre 2009 (2009-12-31), XP055261235, Extrait de l'Internet: URL:http://download.springer.com/static/pd f/73/art%3A10.1007%2Fs12274-009-9025-8.pdf ?originUrl=http://link.springer.com/articl e/10.1007/s12274-009-9025-8&token2=exp=145 9255843~acl=/static/pdf/73/art%253A10.1007 %252Fs12274-009-9025-8.pdf?originUrl=http% 3A%2F%2Flink.springer.com%2Farticle%2F10.1 007%2Fs122 [extrait le 2016-03-29]
- FUPING GAO ET AL: "Pullulan acetate coated magnetite nanoparticles for hyper-thermia: Preparation, characterization and in vitro experiments", NANO RESEARCH, vol. 3, no. 1, 1 janvier 2010 (2010-01-01) , pages 23-31, XP055261219, CN ISSN: 1998-0124, DOI: 10.1007/s12274-010-1004-6

## Description

### DOMAINE DE L'INVENTION

Le domaine de l'invention est celui de nanoparticules synthétiques d'origine bactérienne, modifiées, utilisables en médecine ou en cosmétique.

### ARRIERE PLAN TECHNIQUE

Les bactéries magnétotactiques synthétisent des nanoparticules d'oxyde de fer, appelées magnétosomes, qui possèdent des propriétés remarquables dues d'une part à la présence d'un cœur minéral cristallisé de grande taille, typiquement de l'ordre de 20 à 120 nm de diamètre menant à des propriétés ferrimagnétiques avantageuses, et d'autre part à leur arrangement en chaînes permettant de limiter l'agrégation des magnétosomes. Ces nanoparticules d'origine bactérienne présentent des propriétés magnétiques avantageuses comparées aux nanoparticules issues de synthèse chimique. Par exemple, pour une concentration équivalente en fer et lorsqu'elles sont soumises à l'application d'un champ magnétique alternatif, des suspensions de chaînes de magnétosomes extraites -des bactéries magnétotactiques émettent plus de chaleur que les nanoparticules chimiques couramment utilisées pour l'hyperthermie magnétique telles que les nanoparticules d'oxyde de fer chimiques superparamagnétiques (NOFCS) et les nanoparticules d'oxyde de fer chimiques ferrimagnétiques (NOFCF). Lorsque de telles suspensions sont administrées à des tumeurs du cancer du sein xéno-greffées sous la peau de souris et soumises à plusieurs applications d'un champ magnétique alternatif, cela induit une activité anti-tumorale (Alphandéry et al, AcsNano, V. 5, P. 6279 (2011)). En outre, pour une concentration équivalente en fer, cette activité anti-tumorale est supérieure à celle observée avec les NOFCF ou NOFCS.

Toutefois, ces chaînes de magnétosomes sont issues de bactéries à gram négatif et peuvent donc posséder dans certains cas une concentration élevée en endotoxines ainsi que du matériel biologique difficile à caractériser précisément. C'est donc un objet de la présente invention que de proposer une méthode de fabrication de magnétosomes permettant de surmonter ces problèmes.

Xie, J. et al., Production, modification and bio-applications of magnetic nanoparticles gestated by magnetotactic bacteria (Nano Res. 2, 261-278 (2009)) est une revue relative à l'histoire de la découverte des bactéries magnétotactiques et aux efforts ultérieurs pour élucider les mécanismes derrière la formation du magnétosome. L'accent est mis sur la manière d'utiliser les connaissances acquises grâce aux études fondamentales pour fabriquer des nanoparticules fonctionnelles capables de résoudre de réels problèmes biomédicaux.

### DESCRIPTION

La présente invention découle de la mise en évidence inattendue, par l'(les) inventeur(s), qu'il était possible de remplacer le revêtement périphérique naturel des magnétosomes par un autre revêtement pour aboutir à des nanoparticules synthétiques apyrogènes qui peuvent être utilisées en santé, en diagnostic et/ou en cosmétique.

Ainsi, la présente invention concerne une préparation comprenant au moins une nanoparticule synthétique, dans laquelle la nanoparticule comprend :
- une partie centrale minérale cristallisée comprenant majoritairement un oxyde de fer, synthétisée par un organisme vivant, et
- un revêtement périphérique soit comprenant moins de 10% de matière organique non dénaturée provenant de l'organisme vivant soit ne comprend pas de protéines provenant de l'organisme vivant,
dans lequel ledit organisme vivant est une bactérie magnétotactique.

La préparation selon l'invention peut comprendre au moins 1, 2, 3, 5, 10, 100, 10³, 10⁴, 10⁵, 10⁶, 10⁷, 10⁸, 10⁹ nanoparticules synthétiques contenues dans un volume de 1 µm³, 1 mm³, 1 cm³, ou 1 dm³. Elle peut être une suspension de nanoparticules synthétiques, préférentiellement contenues dans un milieu liquide, ou être un ensemble de nanoparticules synthétiques contenues dans un milieu liquide, solide ou gazeux.

La présente invention concerne également une partie centrale cristallisée comprenant majoritairement un oxyde de fer, synthétisée par un organisme vivant, et ne possédant pas de revêtement.

Une nanoparticule synthétique est une particule dont la taille dans au moins une dimension est inférieure à 10, 5, 2, 5 ou 1 µm, préférentiellement à 750, 500, 400 ou 300 nm, le plus préférentiellement à 200 ou 100 nm, qui est préférentiellement à l'état solide et dont la taille est notamment mesurable au microscope à transmission électronique (MET). Le terme synthétique indique que la nanoparticule a été fabriquée à l'aide d'au moins une étape faisant intervenir l'homme, notamment à l'aide de procédés biologiques ou chimiques.

La nanoparticule synthétique selon l'invention peut posséder au moins une propriété magnétique, telle que :
- être diamagnétique, superparamagnétique, préférentiellement ferrimagnétique ou ferromagnétique,
- posséder au moins un domaine magnétique,
- posséder au moins un moment magnétique non nul,
- posséder une coercivité supérieure à 0,5, 1, 10, 100, ou 1000 Oe à température supérieure à 0, 10, 100, 273, 310, 373, ou 1000 K, ou
- posséder un rapport entre aimantation rémanente et aimantation à saturation supérieur à 0,01, 0,1, 0,2, 0,5, 0,7, 0,9, 0,95, ou 0,99 à température supérieure à 0, 10, 100, 273, 310, 373, ou 1000 K,
ces propriétés étant préférentiellement dues aux propriétés de sa partie centrale.

La nanoparticule synthétique selon l'invention est préférentiellement une nanoparticule possédant un seul domaine magnétique pouvant lui conférer de meilleures propriétés magnétiques par rapport à une nanoparticule possédant plusieurs domaines magnétiques.

La nanoparticule synthétique selon l'invention est préférentiellement une nanoparticule ferrimagnétique ou ferromagnétique, notamment une nanoparticule dont le moment magnétique est stable thermiquement à température physiologique et/ou dont la taille est supérieure à 1, 2, 5, 10, 15, 20, 25, 30, 35, 40, 45, 50, 55, 60, 70, 80, 90, 100, 150, 200, 500 ou 1000 nm.

La nanoparticule synthétique selon l'invention peut être une nanoparticule superparamagnétique, notamment une nanoparticule dont le moment magnétique est instable thermiquement à température physiologique et/ou dont la taille est inférieure à 1, 2, 5, 10, 15, 20, 25, 30, 35, 40, 45, 50, 55, 60, 70, 80, 90, 100, 150, 200, 500 ou 1000 nm.

Le diamètre moyen de la nanoparticule synthétique selon l'invention peut être mesurée au microscope à transmission électronique (MET) ou à l'aide d'une méthode de diffusion de la lumière, dynamique ou non.

La partie centrale de la nanoparticule synthétique comprend majoritairement un oxyde de fer, c'est à dire au moins 10 ou 25%, préférentiellement au moins 50, 75, 95, 97, 99, 99,5 ou 99,9 % d'oxyde de fer. Ce pourcentage en oxyde de fer correspond notamment au nombre d'atomes contenus dans l'oxyde de fer de la partie centrale divisé par le nombre d'atomes contenus dans l'oxyde de fer de la nanoparticule synthétique ou à la masse d'oxyde de fer contenu dans la partie centrale divisée par la masse d'oxyde de fer contenue dans la nanoparticule synthétique. De préférence, l'oxyde de fer est de la maghémite, de la magnétite ou une composition intermédiaire entre la maghémite et la magnétite.

De préférence, l'organisme vivant synthétisant la partie centrale des nanoparticules synthétiques selon l'invention est une cellule eucaryote ou une bactérie, plus préférentiellement une bactérie synthétisant des nanoparticules magnétiques, et encore plus préférentiellement une bactérie magnétotactique.

De manière tout particulièrement préférée, les bactéries selon l'invention appartiennent aux souches magnétotactiques, notamment les *Magnetospirillum magneticum* AMB-1, *Magnetococcus* marinus sp. MC-1, *Magnetovibrio blakemorei* MV-1, MV-2 et MV-4, *Magnetospirillum magnetotacticum* MS-1, *Magnetospirillum gryphiswaldense* MSR-1, *Magnetospirillum magneticum* MGT-1, et *Desulfovibrio magneticus* RS-1.

On notera également que la nanoparticule synthétique selon l'invention se distingue des magnétosomes extraits des bactéries magnétotactiques, habituellement arrangés en chaînes, par l'absence de matière organique provenant des bactéries magnétotactiques dans leur revêtement. Le revêtement ne contient préférentiellement pas de matière organique non dénaturée. La dénaturation est définie ici comme la perte, par une macromolécule biologique (par exemple acide nucléique ou protéine), de sa conformation tridimensionnelle normale. La nanoparticule synthétique peut en effet contenir, dans son revêtement, de la matière organique dénaturée provenant des bactéries magnétotactiques. Cette matière organique dénaturée peut apparaître suite à un traitement destiné à purifier les nanoparticules tel qu'un traitement chimique, thermique, magnétique ou encore une combinaison de ces traitements.

Dans un mode de réalisation, le revêtement contient moins de 50%, 25%, 10%, ou 1% de matière organique non dénaturée provenant des bactéries magnétotactiques, par exemple des lipides, des endotoxines et/ou des protéines non dénaturés provenant des bactéries magnétotactiques. Ce pourcentage de matière organique non dénaturée correspond notamment à la masse de cette matière contenue dans le revêtement divisée par la masse de la nanoparticule synthétique. Cette faible quantité de matière organique issue des bactéries peut être caractérisée par une masse de groupements fonctionnels chimiques présents à la surface de ou dans la partie centrale purifiée, tels que les phosphates ou les amines, qui est inférieure à 1 mg, 0,1 mg, 0,01 mg, 0,001 mg, 0,0001 mg ou 40 ng pour 1 mg en fer total de la partie centrale. Cette faible présence de matière organique non dénaturée peut permettre de réaliser une suspension de nanoparticules synthétiques avec un niveau de pyrogénicité suffisamment faible selon les normes en vigueur, notamment pour permettre une administration à l'Homme ou à l'animal. Dans un mode de réalisation, la préparation selon l'invention contient au moins 2, 5, 10, 20, 30, 50, 100, 150, 200, 500, 10000, 50000, ou 100000 nanoparticules synthétiques qui sont liées l'une à l'autre par du matériau de liaison. Le matériau de liaison peut posséder au moins l'une des propriétés suivantes : i), être constitué, au moins en partie, du(des) même(s) matériau(x) que celui(ceux) qui constitue(nt) le revêtement des nanoparticules synthétiques, ii), lier au moins deux nanoparticules synthétiques entre elles, sur une distance inférieure ou supérieure à 1 mm, 100 µm, 10 µm, 1 µm, 100 nm, 50 nm, 40 nm, 30 nm 20 nm, 10 nm, 5 nm, 2 nm, ou 1 nm, iii), favoriser l'internalisation cellulaire, iv), être chargé positivement ou négativement ou être neutre, v), avoir ou ne pas avoir un effet thérapeutique ou de diagnostic, vi), être suffisamment résistant pour assurer le lien entre au moins deux nanoparticules synthétiques soit lorsque la préparation est dans un milieu, liquide, physique, gazeux ou biologique, soit après une stérilisation soit après un traitement de nature physique, biologique ou chimique, par exemple à l'aide de la solution ou milieu servant à la formulation de la préparation notamment pour l'administration à un organisme, vii), contenir moins de 20%, 15%, 10%, 5%, 2%, 1%, 0,1%, 0,01%, ou 0,001% de matière carbonée provenant de l'organisme vivant, viii), ne pas provenir de l'organisme vivant.

Les nanoparticules synthétiques peuvent être arrangées suivant une figure géométrique qui peut être : une chaîne droite, un cercle, un carré, un rectangle, un triangle, un pentagone, un hexagone, un heptagone, un octogone, un polygone ou un polyèdre. Ces figures peuvent être observées au Microscope Electronique à Transmission.

Un arrangement d'au moins deux nanoparticules synthétiques suivant une figure géométrique peut correspondre à un arrangement de ces nanoparticules ayant : i), des axes cristallographiques orientés dans une direction préférentielle, ii), au moins deux côtés ou faces appartenant à deux nanoparticules synthétiques différentes qui sont parallèles ou perpendiculaire ou qui forment un angle supérieur ou inférieur à 1, 2, 5, 10, 15, 20, 25, 30, 35, 40, 45, 50, 60, 70, 80, 90, 100, 125, 150, 180, 225, 250, 300, 325, 350, ou 379 degrés, iii), un alignement parallèle à un champ magnétique lorsque ces nanoparticules sont soumises à l'application d'un champ magnétique, notamment un champ d'intensité supérieure à 10⁻¹⁰ T, 10⁻⁹ T, 10⁻⁸ T, 10⁻⁷ T, 10⁻⁶ T, 10⁻⁵ T, 10⁻⁴ T, 10⁻³ T, 10⁻² T, 10⁻¹ T, 1 T ou iv), des matériaux de liaison liant au moins deux nanoparticules synthétiques entre elles.

Un arrangement d'au moins deux nanoparticules synthétiques suivant une figure géométrique peut être caractérisé par une variation du potentiel zéta en fonction du pH qui est une variation continue telle qu'une diminution ou augmentation continue, préférentiellement une diminution continue, lorsque le pH augmente de 1 à 14, 2 à 12, 3 à 11, 4 à 10, 5 à 9, ou 6 à 8, où une diminution ou augmentation continue peut correspondre à une variation mesurée entre plus d'1, 2, 3, 4, 5, 6, 7, 8, 9, 10, 11, 12, 13, 14 unités de pH différente(s) qui est soit une diminution soit une augmentation.

Par contraste, un arrangement d'au moins deux nanoparticules synthétiques qui n'est pas suivant une figure géométrique, tel qu'un agrégat, peut être caractérisé par une variation du potentiel zéta en fonction du pH qui est une succession d'au moins 1, 2, 3, 4, 5, 7, 9, 10, 12, 15, 20 diminution(s) et augmentation(s) lorsque le pH augmente de 1 à 14, 2 à 12, 3 à 11, 4 à 10, 5 à 9, ou 6 à 8.

Un arrangement suivant une figure géométrique peut avoir lieu dans un milieu solide, liquide ou gazeux, c'est-à-dire notamment un milieu entourant les nanoparticules synthétiques tel que le milieu utilisé pour la formulation des nanoparticules synthétiques ou celui entourant ces nanoparticules lors de leurs études ou caractérisations.

Selon l'invention, on préfère que lorsque l'organisme vivant synthétisant la partie centrale des nanoparticules synthétiques est une bactérie magnétotactique, les nanoparticules synthétiques selon l'invention comprennent les parties centrales des magnétosomes revêtues d'un composé. De préférence, la partie centrale des nanoparticules synthétiques selon l'invention présente l'une au moins des propriétés suivantes:
(α), une partie cristalline présentant au moins 1, 2, 5, 10, 50, 100, 150 ou 200 plans cristallins, étant préférentiellement sous forme minérale, comprenant majoritairement de l'oxyde de fer, préférentiellement de la maghémite, notamment lorsque la partie centrale est mise en présence d'oxygène, de la magnétite, notamment lorsque la partie centrale n'est pas mise en présence d'oxygène, ou un mélange de maghémite et de magnétite,
(β), un comportement ferrimagnétique ou ferromagnétique, notamment aux températures physiologiques,
(χ), un seul domaine, ou monodomaine, magnétique,
(δ), une microstructure magnétique, pouvant être caractérisée par la présence de lignes de champ magnétique, pouvant être orientées dans une direction préférentielle telle qu'un axe de facile aimantation ou une direction cristallographique de la partie centrale des nanoparticules synthétiques telle que [111], où une telle microstructure magnétique peut dans certaines conditions être observable, notamment par holographie électronique,
(ε), une taille comprise entre 1 nm et 2 µm, 5 nm et 1 µm, 5 et 500 nm, 5 et 250 nm, 5 et 100 nm, 5 et 80 nm, 5 et 60 nm, 10 nm et 1 µm, 10 et 500 nm, 10 et 250 nm, 10 et 100 nm, 10 et 80 nm, 10 et 60 nm, 15 nm et 1 µm, 15 et 500 nm, 15 et 250 nm, 15 et 100 nm, 15 et 80 nm, 15 et 60 nm, 20 nm et 1 µm, 20 et 500 nm, 20 et 250 nm, 20 et 100 nm, 20 et 80 nm ou 20 et 60 nm, (φ), une taille supérieure à 1, 2, 5, 10, 15, 20, 25, 30, 35 ou 40 nm,
(γ), une taille inférieure à 2000, 1000, 500, 400, 300, 200, 150, 120, 100, 95, 90, 80, 75, 70, 65, 60, 55, 50, 45, 40, 35, 30, 25, 20, 15, 10 ou 5 nm,
(η), la présence possible d'un potentiel zéta, d'une charge, d'une charge de surface,
( 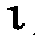 ), un point isoélectrique, préférentiellement acide de pH inférieur ou égal à 7, 6, 5, 4, 3, 2 ou 1,
( 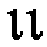 ), un point isolélectrique d'autant plus proche de 7 qu'il reste peu de matière organique à la surface de la partie centrale ; lorsqu'il reste peu de matière organique, le point isoélectrique peut être compris entre pH 6,5 et pH 7,5, entre pH 6,2 et pH 7,1, ou entre pH 6,1 et pH 7,1.

Comme l'homme du métier le comprendra bien, le potentiel zéta, la charge, la charge de surface dépendent habituellement du pH, de la salinité, de l'espèce bactérienne synthétisant la partie centrale des nanoparticules synthétiques, de l'état d'agrégation, du pourcentage de matière organique présent à la surface de ces parties centrales. Ils peuvent : (1), être positifs à pH acide, à pH inférieur ou égal à 7, 6, 5, 4, 3, 2 ou 1, (2), être négatifs à pH basique, à pH supérieur ou égal à 13, 12, 11, 10, 9, 8, 7, (3), varier en fonction du pH dans un intervalle compris entre -10 et 10 mV, -20 et 20 mV, -30 et 30 mV, -40 et 40 mV, -50 et 50 mV, -60 et 60 mV, -70 et 70 mV, -80 et 80 mV, -90 et 90 mV, -100 et 100 mV, -150 et 150 mV ou -200 et 200 mV, (4), varier d'autant plus en fonction du pH qu'il reste peu de matière, préférentiellement peu de matière carbonée, à la surface des parties centrales des nanoparticules synthétiques.

De préférence, la partie centrale des nanoparticules synthétiques comprend moins de 50, 25, 10, 5, 2, 1, 0,5, 0,2, 0,1, 0,05, 0,01 ou 0,001% de matière organique. Comme on l'entend ici, ce pourcentage de matière organique représente le pourcentage en masse ou en nombre d'atomes ou en volume de n'importe quel élément chimique ou combinaison d'éléments chimiques contenus dans la matière organique tels que le carbone, l'azote, le phosphore. Ce pourcentage peut être mesuré à l'aide d'appareils d'analyse élémentaire tels que les analyseurs de carbone organique total (COT) ou de carbone, hydrogène, azote, soufre, oxygène (CHNS/O).

Dans un mode de réalisation, le revêtement selon l'invention, aussi appelé revêtement périphérique, est de la matière à l'état solide, liquide ou gazeux, préférentiellement à l'état solide, qui entoure la partie centrale, notamment sous forme de couche, cristallisée ou non, notamment sur une distance mesurée à partir du centre de la partie centrale qui est inférieure à 100 µm, 10 µm, 5 µm, 1 µm, 500 nm, 250 nm, 100 nm, 10 nm, ou 1 nm. Ce revêtement peut notamment servir à stabiliser la partie centrale ou à lier les nanoparticules synthétiques les unes aux autres.

Le revêtement peut posséder au moins une propriété commune avec la partie centrale ou au moins une propriété différente de la partie centrale.

Selon l'invention, le revêtement périphérique ne comprend de préférence pas de matière non dénaturée provenant de l'organisme vivant. Il peut contenir moins de 50, 40, 30, 20, 10, 5, 4, 3, 2, 1, 0,1, 0,05, 0,01, 0,001 ou 0,0001% de cette matière. Ce pourcentage peut être défini comme étant le rapport entre le nombre des atomes provenant de l'organisme vivant contenus dans le revêtement divisé par le nombre total des atomes contenus dans le revêtement ou le rapport entre la masse des atomes provenant de l'organisme vivant contenus dans le revêtement divisé par la masse totale des atomes contenus dans le revêtement. Ce pourcentage peut correspondre à un pourcentage de matière carbonée ou organique.

La nanoparticule synthétique, selon l'invention, est apyrogène, où l'apyrogènicité peut être définie par une mesure de quantité d'endotoxines par milligramme de nanoparticule synthétique, préférentiellement contenu dans 1 millilitre de la préparation, qui est inférieure ou égale à 10⁹, 10⁸, 10⁷ ou 10⁶, préférentiellement à 10⁴ ou 10³, le plus préférentiellement à 100, 50, 10, 5 ou 1 unité d'endotoxine (UE). Comme l'homme du métier le sait bien, une UE peut correspondre à une quantité de 100 pg par mL d'endotoxines issues d'E-Coli.

De préférence, la nanoparticule synthétique est apyrogène lorsque son revêtement ou sa partie centrale est apyrogène, préférentiellement lorsque son revêtement et sa partie centrale sont apyrogènes, où l'apyrogènicité peut dans ce cas être définie par une mesure de quantité d'endotoxines par milligramme de la partie centrale et/ou du revêtement de la nanoparticule synthétique, préférentiellement contenu(s) dans 1 millilitre de la préparation, qui est inférieure ou égale à 10⁹, 10⁸, 10⁷ ou 10⁶, préférentiellement à 10⁴ ou 10³, le plus préférentiellement à 100, 50, 10, 5 ou 1 unité d'endotoxine (UE).

Comme on l'entend ici, une préparation est apyrogène lorsque les substances autres que les nanoparticules synthétiques contenues dans la préparation, telles que les substances contenues dans l'excipient et/ou le solvant de la préparation et/ou la matrice entourant les nanoparticules synthétiques sont apyrogènes.

De préférence, la préparation apyrogène est caractérisée par un pourcentage en endotoxines inférieur ou égale à 50, 20, 1, 10⁻¹, 10⁻², 10⁻³, 10⁻⁴, 10⁻⁵, 10⁻⁶, 10⁻⁷, 10⁻⁸ ou 10⁻⁹%, où ce pourcentage peut être : (i), la masse d'endotoxines divisée par la masse totale de la préparation, (ii), le volume occupé par les endotoxines divisé par le volume total de la préparation, (iii), le nombre d'atomes contenus dans les endotoxines de la préparation divisé par le nombre total d'atomes contenu dans la préparation.

De préférence, la préparation apyrogène est caractérisée par une quantité d'endotoxines inférieure ou égale à 10⁹, 10⁸, 10⁷ ou 10⁶, préférentiellement à 10⁴ ou 10³, le plus préférentiellement à 100, 50, 10, 5 ou 1 UE ou UE par milligramme d'oxyde de fer ou UE par millilitre ou UE par milligramme d'oxyde de fer par millilitre.

Dans un mode de réalisation, la préparation apyrogène est un médicament administré à un organisme tel qu'un individu ou un animal. Elle comprend alors de préférence une quantité en endotoxines inférieure à : (i), 5 UE par kilogramme de poids corporel de cet organisme par heure d'administration pour une administration par voie non intrathécale et, (ii), 0,2 UE par kilogramme de poids corporel de cet organisme par heure d'administration pour une administration par voie intrathécale.

Dans un mode de réalisation, la préparation apyrogène est un dispositif médical, notamment invasif. Elle contient alors de préférence une quantité en endotoxines, de préférence mesurée à la surface du dispositif médical, qui est inférieure à : (i), 0,5 UE par millilitre de la préparation lorsque cette dernière n'est pas en contact avec le liquide céphalo-rachidien et (ii), 0,02 UE par millilitre de la préparation lorsque cette dernière est en contact avec ce liquide.

De préférence, la préparation apyrogène selon l'invention contient une quantité d'endotoxines conforme aux normes réglementaires en vigueur applicables au médicament, au dispositif médical ou au produit cosmétique, en particulier conforme à la pharmacopée, le plus préférentiellement à la(aux) pharmacopée(s) européenne et/ou américaine.

La préparation apyrogène peut éventuellement être administrée à un organisme en un temps d'administration supérieur ou égal auxdites valeurs suivantes: 1, 30 ou 60 seconde(s), 1, 30 ou 60 minute(s), 1, 15 ou 24 heure(s), 1 ou 5 jour(s), 1 ou 4 semaine(s). Dans le cas où la préparation apyrogène est administrée à un organisme en un temps d'administration supérieur ou égal auxdites valeurs, la quantité d'endotoxines contenue dans la préparation peut être plus importante que dans le cas où cette même préparation est administrée en un temps d'administration inférieur auxdites valeurs.

De préférence, la quantité d'endotoxines dans la préparation est mesurée par un test de lysat d'amibiocyte de limule (LAL). Comme l'homme du métier le comprendra, on préférera s'assurer que les nanoparticules n'interfèrent pas avec le test, notamment en mesurant un taux de recouvrement. Ce taux peut être défini comme égal à Cₜₒₜₐₗ/C₁+C₂, où Cₜₒₜₐₗ est la concentration en endotoxines des suspensions de nanoparticules synthétiques mélangées à une quantité connue d'endotoxines de par exemple 0,5 UE/mL Dans cet exemple, C₁ est la concentration en endotoxines dans les différentes suspensions de nanoparticules synthétiques et C₂ = 0,5 EU/mL. Il peut être considéré qu'il n'y a pas d'interférences lorsque ce taux de recouvrement est supérieur ou égal à 10 ou 30, préférentiellement à 50, 100 ou 150%.

La pyrogénicité de la préparation selon l'invention peut être évaluée à l'aide d'un test de pyrogénicité sur le lapin, notamment suivant la norme ISO 10993-11, le chapitre 151 de la pharmacopée américaine ou la pharmacopée européenne. Ce test peut être réalisé en administrant la préparation en intraveineuse chez 1 ou plusieurs lapins, préférentiellement 3, préférentiellement à une dose de nanoparticules synthétiques qui est typiquement mais pas obligatoirement supérieure ou égale à 1 mg. Notamment dans le cas où la préparation est un dispositif médical, une telle dose semble adéquate, car supérieure aux 6 cm² par ml ou mg de nanoparticules synthétiques, notamment recommandés par la norme ISO10993-12. En effet, considérant la surface d'une nanoparticule synthétique cubique de 50 nm de côté qui est de 15.10⁻¹¹ cm² et qu'un mg de nanoparticule synthétique composée majoritairement de maghémite contient 1.6 10⁻¹² nanoparticules synthétiques, il peut être estimé qu'1 mg de nanoparticules synthétiques possède une surface de 94 cm², supérieure aux 6 cm². L'absence de pyrogénicité de la préparation est ensuite mise en évidence soit en mesurant la somme des élévations de température chez trois lapins qui ne doit pas excéder 10, 5, 2 ou 1°C, préférentiellement 2,65°C en conformité avec la pharmacopée européenne, soit en mesurant l'élévation de température chez chaque lapin qui ne doit pas excéder 10, 5, 2, 1 ou 0,1°C, préférentiellement 0,5°C en conformité avec la pharmacopée américaine soit en suivant les recommandations d'une ou plusieurs pharmacopées, notamment les pharmacopées européennes et américaines.

De préférence, la préparation selon l'invention est apyrogène ou non-immunogène. Notamment, l'immunogénicité ou la pyrogénicité peut être causée par des substances biologiques non minérales, des lipides, protéines, enzymes, ADN, ou ARN, que ces substances soient fabriquées par un autre organisme vivant que l'homme ou qu'elles soient synthétisées au moins en partie par l'homme.

De préférence, la préparation, selon l'invention, est apyrogène ou non-immunogène lorsqu'elle contient moins de 10⁹, 10⁸, 10⁷, 10⁶, 10⁵, 10⁴, 10³, 10², 10¹, ou 1 molécule(s) biologique(s) non minérale(s), telles que protéine(s), lipide(s), enzyme(s), ADN, ou ARN.

Dans un mode de réalisation, la préparation, selon l'invention, peut être considérée comme apyrogène ou non immunogène lorsqu'elle n'induit pas en elle-même de réponse significative du système immunitaire, c'est à dire qu'elle n'a pas d'activité pharmaceutique ou médicale significative ; la préparation n'a notamment pas d'activité antitumorale en elle-même. Comme l'homme du métier le comprendra bien, la préparation peut avoir une activité pharmaceutique ou médicale, telle qu'une activité antitumorale lorsqu'elle est soumise à l'application d'un rayonnement, tel qu'un champ magnétique, préférentiellement un champ magnétique alternatif. Dans un mode de réalisation, la préparation, la nanoparticule synthétique, sa partie centrale et/ou son revêtement peut(peuvent) être apyrogène(s) et immunogène(s) ou être apyrogène(s) et non immunogène(s). La pyrogénicité peut être considérée comme une immunogénicité causée par des endotoxines.

De préférence, les nanoparticules synthétiques selon l'invention s'agrègent peu, possèdent une distribution homogène dans un milieu donné, préférentiellement dans la préparation et/ou dans l'organisme où elles sont administrées. Cette distribution homogène est caractérisée par: (i), la présence de ces nanoparticules synthétiques dans 0,1, 1, 5, 10, 25 ou 50% de ce milieu, où ce pourcentage peut être défini comme étant le volume occupé par les nanoparticules synthétiques divisé par le volume total du milieu considéré, (ii), une sédimentation des nanoparticules synthétiques se produisant en plus d'une heure, préférentiellement en plus d'une semaine, le plus préférentiellement en plus de 15 jours, (iii), une sédimentation des nanoparticules synthétiques se produisant en un temps supérieur au temps d'administration de la préparation dans un organisme. La sédimentation des nanoparticules synthétiques peut être révélée par la présence d'agrégats qui sédimentent, préférentiellement dans un liquide, et qui sont visibles à l'œil nu, au microscope optique ou par des mesures spectroscopiques, notamment par absorption.

De préférence, les nanoparticules synthétiques selon l'invention sont stables en suspension ou la préparation selon l'invention est stable. La stabilité est définie comme étant la capacité des nanoparticules synthétiques à rester en suspension sans sédimenter. La stabilité peut notamment être mesurée par des mesures d'absorption, préférentiellement par des mesures de variation d'absorption au cours du temps à une longueur d'onde où les nanoparticules synthétiques absorbent, typiquement comprise entre 400 nm et 600 nm, le plus typiquement égale à 480 nm, à une longueur d'onde supérieure à 1, 50, 100, 200, 300, 400, 450, 500, 600 ou 800 nm, 1, 5 ou 10 µm, à une longueur d'onde inférieure à 10, 5 ou 1 µm, 800, 600, 500, 450, 400, 300, 200, 100, 50 ou 1 nm. De préférence, la stabilité est telle que la densité optique ou l'absorption de la suspension de nanoparticules synthétiques ne diminue pas de plus de 50, 25, 10, 5, 2, 1 ou 0,1 %, où ce pourcentage de diminution peut être défini comme étant égal à : [abs(t₀)-abs(t)]/abs(t₀), où abs(t₀) et abs(t) sont les absorptions mesurées respectivement en début et en fin de mesure. Cette diminution peut notamment être mesurée au cours du temps pendant une période de plus d'1, 5, 15, 25, 45 ou 60 secondes, 2, 5, 10, 30, 45 ou 60 minutes, 2, 5, 10, 50, 100 ou 1000 fois la durée nécessaire pour administrer la suspension de nanoparticules synthétiques à un organisme. Elle peut être mesurée juste après avoir réalisé la préparation, quelques jours, quelques semaines, quelques mois, quelques années après avoir réalisé la préparation. Elle est de préférence mesurée après avoir homogénéisé la préparation, notamment à l'aide de sonication, de vibration, d'agitation.

Selon un mode de réalisation, la préparation selon l'invention est stable pour une concentration en fer de nanoparticules synthétiques supérieure à 0,01, 0,05, 1, 5, 10, 30, 50, 100, 150, 200, 250, 300, 400, 500, 600, 700, 800 ou 900 mg/mL, 1, 2, 5 ou 10 g/mL.

Dans un mode de réalisation, la nanoparticule synthétique possède une homogénéité de distribution, une stabilité, qui est(sont) supérieure(s) à celle(s) des nanoparticules synthétiques extraites d'un organisme vivant, préférentiellement d'une bactérie magnétotactique ou à celle(s) des parties centrales des nanoparticules synthétiques ou des NOFCS ou des NOFCF.

Dans un mode de réalisation, la nanoparticule synthétique peut posséder :
- Une propriété commune avec sa partie centrale, préférentiellement la propriété (α), (β), (χ), (δ), (η) (voir page 5-6),
- Une taille supérieure de 0,01, 0,05, 0,1, 0,2, 0,5, 1, 2, 3, 4 5, 6, 7, 8, 9, 10, 15, 20, 50, 75, 100, 250 ou 500 nm, 1, 5, 10, 50 ou 100 µm à la taille de sa partie centrale et/ou de son revêtement,
- Un point isoélectrique, notamment un point isoélectrique différent de celui de sa partie centrale et/ou de son revêtement,
- Un potential zéta, une charge, une charge de surface, notamment n'importe lequel de ces trois paramètres et/ou de leur variation en fonction du pH qui est(sont) différent(s) de celui(ceux) de sa partie centrale et/ou de son revêtement,
- Une apyrogénicité qui est inférieure, supérieure ou égale à celle de sa partie centrale et/ou de son revêtement.

Dans un mode de réalisation, le revêtement selon l'invention favorise l'accrochage de substances sur les nanoparticules synthétiques, permet de limiter la toxicité et résulte en une organisation spécifique telle que : (i), un arrangement en chaînes, c'est-à-dire un arrangement d'au moins deux nanoparticules synthétiques liées l'une à l'autre avec un possible alignement des axes cristallographiques ou des lignes de champ de ces nanoparticules, (ii), un arrangement suivant une figure géométrique, (iii), ordonnée et caractérisée par la présence d'un motif géométrique, tel qu'un cercle, un rectangle, un losange, un carré, une éclipse. Une telle organisation peut être mise en évidence par des mesures MET, notamment en déposant une goutte de la préparation de nanoparticules synthétiques à une concentration adéquate pour pouvoir observer les arrangements des nanoparticules synthétiques dans leurs ensembles.

Dans un mode de réalisation, le revêtement n'a pas pour origine l'organisme qui synthétise la partie centrale des nanoparticules synthétiques, ce qui peut faciliter sa caractérisation, notamment lorsque la structure ou composition du revêtement est connue, simple, identifiable ou aisément caractérisable.

Dans un mode de réalisation, le revêtement est une substance apyrogène.

Dans un mode de réalisation, le revêtement comprend au moins un composé capable d'établir des interactions faibles ou des liaisons covalentes avec la partie centrale des nanoparticules synthétiques, notamment l'oxyde de fer.

Dans un mode de réalisation, le revêtement comprend au moins un composé capable d'être chimisorbé ou physisorbé sur la partie centrale de la nanoparticule synthétique.

Dans un mode de réalisation, le revêtement comprend au moins un composé capable d'établir des interactions ou des liaisons avec les ions Fe²⁺ ou Fe³⁺, hydroxyles OH⁻, oxydes O²⁻, des défauts cristallins de la partie centrale, pouvant se trouver dans ou à la surface de la partie centrale des nanoparticules synthétiques.

Dans un mode de réalisation, le revêtement comprend au moins un composé, un atome, un ion, ou une fonction chimique telle qu'une fonction acide, acide carboxylique, acide phosphorique, ou acide sulfonique, où le composé, atome ou ion compris dans le revêtement est capable d'établir des interactions ou des liaisons avec la partie centrale ou avec au moins un atome de la partie centrale, une fonction chimique de la partie centrale, un ion de la partie centrale tel que l'ion Fe²⁺, Fe³⁺, hydroxyle OH⁻, oxyde O²⁻ ou un défaut cristallin de la partie centrale.

L'atome, la fonction chimique ou l'ion de la partie centrale peut se trouver dans ou à la surface de la partie centrale des nanoparticules synthétiques.

Dans un mode de réalisation, le revêtement est choisi parmi les substances qui confèrent aux nanoparticules synthétiques des propriétés de chauffage meilleures que celles des NOFCS ou NOFCF. On préfère ainsi que le revêtement comprenne des substances qui sont de bons conducteurs thermiques.

Dans un mode de réalisation, le revêtement est choisi parmi les composés qui confèrent aux nanoparticules synthétiques une organisation ou des propriétés d'assemblage qui favorisent les effets d'un rayonnement ou d'un champ magnétique tel qu'un champ magnétique alternatif sur ces nanoparticules synthétiques. Les effets d'un rayonnement ou d'un champ magnétique peuvent notamment être des mouvements, des vibrations, des rotations, ou des translations de ces nanoparticules.

Dans un mode de réalisation, le revêtement possède une épaisseur qui est inférieure au diamètre moyen de la partie centrale des nanoparticules synthétiques, inférieure à la moitié, au quart de ce diamètre, à 10, 5, 2,5 ou 1 µm, 500, 400, 300, 200, 100, 90, 80, 70, 60, 50, 40, 30, 20, 10, 5, 4, 3, 2, 1 ou 0,5 nm. Une telle épaisseur peut notamment permettre de limiter la toxicité.

Dans un mode de réalisation, le revêtement possède une épaisseur typiquement supérieure au diamètre moyen de la partie centrale des nanoparticules synthétiques, à la moitié, au quart de ce diamètre, à 0,1, 0,5, 1, 2, 4, 8, 10, 15, 20 ou 25 nm. Une telle épaisseur peut notamment permettre de lier les nanoparticules synthétiques entre elles ou d'éviter la formation d'agrégats. Avantageusement, la présence d'un revêtement suffisamment épais permet d'éviter que les nanoparticules synthétiques ne se collent les unes aux autres, notamment sous l'effet des forces magnétiques qu'elles exercent les unes sur les autres et dont l'intensité peut être d'autant plus forte que ces nanoparticules synthétiques sont proches les unes des autres.

Dans un mode de réalisation, le pourcentage de variation de l'épaisseur du revêtement est défini comme étant la plus petite divisée par la plus grande épaisseur du revêtement pouvant être mesurées sur la(les) nanoparticule(s) synthétique(s) présente(s) dans la préparation, notamment à l'aide du MET. Dans un premier cas, l'épaisseur du revêtement est homogène. Le pourcentage de variation de l'épaisseur est alors de préférence inférieur à 10⁶, 10⁵, 10⁴, 10³, 500, 100, 50, 10, 5, 1 ou 0,1%. Une épaisseur homogène peut notamment permettre une distribution homogène des nanoparticules synthétiques. Dans un deuxième cas, l'épaisseur du revêtement est inhomogène. Le pourcentage de variation de l'épaisseur est alors supérieur à 0,1, 1, 5, 10, 50, 100, 500, 10³, 10⁴, 10⁵ ou 10⁶%. Une épaisseur inhomogène peut notamment résulter en une distribution de forces magnétiques d'intensité variable.

Dans un mode de réalisation, le revêtement possède une teneur en fer inférieure ou égale à 1, 2, 5, 10, 10², 10³, 10⁴, 10⁵ ou 10⁶ fois celle de la partie centrale des nanoparticules synthétiques. Dans un autre mode de réalisation, le revêtement possède une teneur dans au moins un autre atome que le fer et l'oxygène qui est supérieure ou égale à 1, 2, 5, 10, 10², 10³, 10⁴, 10⁵ ou 10⁶ fois celle de la partie centrale des nanoparticules synthétiques.

Dans un mode de réalisation, le revêtement est un surfactant induisant une variation de tension de surface à la surface des nanoparticules synthétiques, supérieure à 10⁻⁴ , 10⁻³, 10⁻², 10⁻¹, 1, 5, 10, 20, 50 ou 100% où ce pourcentage est défini comme étant égal à TS(PC)-TS(NS)/TS(PC), où TS(PC) et TS(NS) sont les tensions de surface respectives des parties centrales des nanoparticules et des nanoparticules synthétiques.

Dans un mode de réalisation, le revêtement comprend des composés carbonés.

Dans un mode de réalisation, le revêtement comprend au moins un composé sélectionné dans le groupe constitué d'un chélatant, d'une molécule amphiphatique, d'un polymère polarisé ou chargé, d'un oxyde de métal ou de silicium, d'un hydroxyde de métal ou de silicium, d'un acide, d'un dérivé acide, basique, oxydé, réduit, neutre, chargé positivement, chargé négativement de ces composés, et d'une combinaison de plusieurs de ces composés ou de ses dérivés.

Dans un mode de réalisation, le revêtement comprend au moins un composé sélectionné dans le groupe constitué d'un polysaccharide, d'un acide gras, d'un phospholipide, d'un polymère d'acides aminés, de silice polymérique ou non et d'un polymère aliphatique aminé, d'un dérivé acide, basique, oxydé, réduit, neutre, chargé positivement, chargé négativement de ces composés, et d'une combinaison de plusieurs de ces composés ou de ses dérivés.

Dans un mode de réalisation, le revêtement ne comprend pas de phospholipides ou de protéines ou d'ARN ou d'ADN ou de composés d'origine bactérienne, cellulaire ou biologique ou de composé provenant d'une bactérie magnétotactique.

Dans un mode de réalisation, le revêtement comprend au moins une fonction sélectionnée dans le groupe constitué des fonctions acides phosphoriques, acides carboxyliques, acides sulfoniques, esters, amides, cétones, alcools, phénols, thiols, amines, éthers, sulfures, anhydrides d'acides, halogénures d'acyles, amidines, nitriles, hydropéroxydes, imines, aldéhydes, peroxydes, un dérivé acide, basique, oxydé, réduit, neutre, chargé positivement, chargé négativement de ces composés, et d'une combinaison de plusieurs de ces composés ou de ses dérivés.

Dans un mode de réalisation, le revêtement selon l'invention est choisi parmi les substances stérilisables, préférentiellement par autoclavage, biocompatibles, biodégradables, qui n'induit pas d'action métabolique, immunologique, cytotoxique, pharmacologique, injectable par voie intraveineuse et/ou immunologique. Une telle substance peut être le povidone, le PEG 400, le poloxamer 188, le dextran, le phosphatidylcholine, le dipalmitoyl-sn-glycero-3-phosphatidylcholine, ou un dérivé de ces substances.

Le type de revêtement peut être choisi en fonction des paramètres suivants:
(i) le mode d'administration des nanoparticules synthétiques : par exemple, pour une administration intraveineuse, un revêtement qui permet d'éviter que les macrophages ne capturent les nanoparticules synthétiques, tel que du PEG ou du dextran peut éventuellement être choisi,
(ii) l'internalisation cellulaire : pour la favoriser, un revêtement avec une charge positive tel que la poly-L-lysine peut «éventuellement» être choisi.

Dans un mode de réalisation, la préparation selon l'invention peut être utilisée à titre de médicament ou d'agent de diagnostic, notamment dans le cadre du traitement d'une tumeur, par exemple par hyperthermie magnétique.

Dans un mode de réalisation, une intervention ou opération médicale, vétérinaire ou cosmétique à l'aide de cette préparation fait intervenir au moins l'une des séquences suivantes, préférentiellement dans l'ordre chronologique indiqué:
(i) l'administration de la préparation à un organisme, notamment par voie locale, cutanéo-muqueuse, entérale, parentérale, intratumorale, intraveineuse, intraartérielle.
(ii) le ciblage des nanoparticules synthétiques vers une partie d'un organisme, tel qu'un organe, une tumeur, un vaisseau sanguin,
(iii) Le traitement ou la détection de la partie de cet organisme.

Dans un mode de réalisation, la préparation selon l'invention peut être utilisée pour les applications cosmétiques.

Dans un mode de réalisation, la préparation selon l'invention est administrée à une concentration supérieure à la concentration maximale à laquelle une suspension contenant des chaînes de magnétosomes extraites des bactéries magnétotactiques peut être administrée, préférentiellement 14 mg/mL en fer. Elle peut également être administrée à une concentration en fer supérieure ou égale à 0,01, 0,05, 1, 5, 10 ou 15 mg/mL, préférentiellement à 25, 50, 100 ou 150 mg/mL, le plus préférentiellement à 200, 300, 400, 500, 600, 700, 800 ou 900 mg/mL, 1, 2, 5 ou 10 g/mL. Cela peut être rendu possible par la plus grande solubilité ou la moindre sédimentation des nanoparticules synthétiques comparée à celle des chaînes de magnétosomes extraites des bactéries magnétotactiques et/ou à celle des NOFCS et/ou à celle des NOFCF.

Dans un mode de réalisation, la préparation selon l'invention peut être administrée à une concentration en fer inférieure ou égale à 1 kg/mL, 500, 250, 100, 50, 10, 5, 2 ou 1 g/mL, préférentiellement à 900, 700, 500 ou 400 mg/mL, le plus préférentiellement à 300, 200, 150, 100, 10, 1 ou 0,1 mg/mL.

Dans un mode de réalisation, le traitement peut être réalisé par application d'un champ magnétique alternatif, une technique couramment dénommée hyperthermie magnétique, alors que la détection peut se faire notamment par imagerie à résonnance magnétique (IRM).

Dans un mode de réalisation, l'invention porte sur une composition pharmaceutique ou médicament comprenant, à titre de principe actif, une préparation telle que décrite précédemment et éventuellement au moins un véhicule pharmaceutiquement acceptable.

Dans un mode de réalisation, l'invention porte sur un dispositif médical comprenant la préparation selon l'invention.

Dans un mode de réalisation, l'invention porte sur une composition de diagnostic comprenant la préparation selon l'invention.

Dans un mode de réalisation, l'invention porte sur une composition cosmétique comprenant à titre de principe actif cosmétique la préparation selon l'invention.

Dans un mode de réalisation, l'invention porte sur une méthode de traitement d'une tumeur d'un individu ou d'un animal dans laquelle on administre une quantité thérapeutiquement active d'une préparation selon l'invention.

Dans un mode de réalisation, l'invention porte sur un procédé de fabrication d'une préparation telle que décrite précédemment, comprenant les séquences suivantes :
(i), à partir d'une préparation de nanoparticules synthétisées par un organisme vivant comprenant une partie centrale minérale cristallisée composée majoritairement d'oxyde de fer et un revêtement périphérique biologique, isoler la partie centrale minérale ;
(ii), traiter la préparation obtenue de manière à couvrir la partie centrale par un revêtement périphérique ;
(iii), éventuellement stériliser la préparation, préférentiellement après la séquence (i), possiblement après la séquence (ii).

Dans un mode de réalisation, la partie centrale des magnétosomes est entourée ou non de composé(s) ou substance(s) n'appartenant pas à cette partie centrale.

Dans un mode de réalisation, les magnétosomes traités sont associés à des magnétosomes ayant subi un traitement, notamment suite à la séquence de fermentation bactérienne.

Dans un mode de réalisation, les séquences (i) et/ou (ii) peut(peuvent) faire intervenir :
(a), un procédé chimique de purification, appelé ledit procédé chimique de purification, dont l'objectif est notamment d'enlever par une méthode chimique tout ou partie de la matière entourant les parties centrales des magnétosomes. Il peut consister à mélanger n'importe quelle suspension des séquences (i) ou (ii) avec un solution chimique, appelée ladite solution chimique, préférentiellement à une concentration supérieure à 0,001, 0,01, 0,1, 1, 10 ou 100 µM, 1, 10 ou 100 mM, 1 M, préférentiellement à une concentration inférieure à 1000, 100, 50, 10, 5, 2 ou 1 M, 500, 100, 50, 10, 5 ou 1 mM, 100, 50, 10, 5 ou 1 µM. Cette solution chimique peut être une solution hypo-osmotique, un tampon de lyse, une solution permettant de désorber ou détacher les magnétosomes de certaines surfaces, d'enlever toute matière, notamment organique, ne se trouvant pas dans ou à la surface de la partie centrale des magnétosomes, d'enlever des restes de détergents. Cette solution chimique peut contenir les substances chimiques suivantes: (i) des dénaturants chimiques tels que l'hydroxyde de sodium, l'hydroxyde de potassium ou des solutions de pH neutres, acides ou basiques, (ii), des solvants organiques tels que le toluène, l'éther, l'alcool le phényléthyl, le diméthylsulfoxyde (DMSO), le benzène, le méthanol, le chloroforme, (iii), des agents chaotropiques, tels que l'urée, le phénol, la guanidine, le chlorure de guanidium, le thiocyanate de guanidium qui sont notamment capables d'amener des composés hydrophobiques dans des solutions aqueuses en modifiant la structure de l'eau, (iv), des agents chélatants tels que l'éthylène diamine tétra-acétique (EDTA), (v), des substances utilisées lors de la dépyrogénation telles que la soude, l'urée, l'eau oxygénée, permettant d'enlever ou de neutraliser les endotoxines, (vi), des antibiotiques, tels que les thionines, (vii), des surfactants tels que le Triton, le Brij, le Duponal, (viii), des agents réducteurs tels que le dithiothréitol (DTT), le thioglycolate, le β mercaptoéthanol, pouvant notamment permettre de casser les liaisons ou ponts disulfure, (ix), des détergents, composés avec un groupement hydrocarboné hydrophobe et une extrémité chargée hydrophile, (x), des tensioactifs. Parmi les détergents qui peuvent être utilisés se trouvent les détergents ioniques, cationiques ou anioniques, les détergents non ioniques, les détergents zwitterioniques, les chaotropes, le sodium dodecyl sulfate (SDS), le desoxycholate, le cholate, le triton, en particulier le triton XI00, le n-Dodécylβ-D-maltoside (DDM), la digitonine, le tween 20, le tween 80, le 3-[(3-cholamidopropyl)dimethylamino]-1-propanesulfonate (CHAPS), l'urée, la guanidine hydrochloride, le nonyl phenoxypolyethoxylethanol (NP-40). Ladite solution chimique peut aussi contenir de l'eau ou n'importe laquelle(lesquelles) du(des) dérivé(s) de la(des) substance(s) chimique(s) précitée(s) ;
(b), un procédé biologique de purification, appelé ledit procédé biologique de purification, dont l'objectif est notamment d'enlever à l'aide d'un procédé biologique tout ou partie de la matière entourant la partie centrale des magnétosomes. Il peut notamment faire intervenir un organisme vivant ou une substance issue d'un tel organisme telle qu'une enzyme, notamment une enzyme lytique, un phage, une protéine telle que la protéase, la mannase ;
(c), un procédé chimique de revêtement, appelé ledit procédé chimique de revêtement, consistant à mélanger la suspension contenant les parties centrales des magnétosomes à n'importe quelle substance ou combinaison de substances utilisée(s) pour le revêtement de la partie centrale des magnétosomes, où cette(ces) substance(s) est(sont) appelée(s) la(les)dite(s) substance(s) de revêtement ;
(d), un procédé thermique, appelé ledit procédé thermique, consistant à faire subir à n'importe quelle suspension obtenue lors des séquences (i) ou (ii) un gradient de température, notamment supérieur ou égal à 10, 25, 50, 100, 150, 200, 300, 500, 1000, 2000, 3000 ou 5000°C et/ou à mettre n'importe quelle suspension obtenue lors des séquences (i) et/ou (ii) à une température, préférentiellement inférieure ou égale à 100, 50, 10, 0, -10, -40, -70, -77, -150, -250°C, 50, 30, 10 ou 1 K, préférentiellement supérieure ou égale à 1, 5, 25, 50K, -250, -70, -77, -50, -20, 0, 5, 20, 40, 100, 200, 500, 1000, 2000, 4000 ou 5000°C.
(e), un procédé mécanique, appelé ledit procédé mécanique, qui consiste à appliquer à n'importe quelle suspension obtenue lors des séquences (i) et/ou (ii) : (1), une pression, préférentiellement supérieure à 1, 10, 100, 500, 10³, 10⁴, 10⁵, 10⁶, 10⁷, 10⁸ ou 10⁹ atmosphère(s), préférentiellement inférieure à 10⁹, 10⁸, 10⁷, 10⁶, 10⁵, 10⁴, 10³, 500, 100, 10 ou 1 atmosphère(s), notamment à l'aide d'un appareil tel qu'un homogénéisateur de haute pression, une presse de French, une bombe à disruption ou un moulin à bille soit, (2), une sonication, notamment à une puissance supérieure à 0,01, 0,1, 0,5, 1, 2, 5, 7, 10, 20, 40, 100, 500 ou 1000 Watt, à une puissance inférieure à 1000,500, 100, 40, 20, 10, 7, 5,2, 1, 0,5, 0,1 ou 0,01 Watt, où la durée de sonication est préférentiellement de plus d'1, 2, 5, 10, 20, 30, 45 ou 60 seconde(s), de plus de 2, 5, 10, 20, 30, 45, 60 ou 120 minutes, de plus de 2, 3, 4 ou 5 heures, préférentiellement de moins de 60, 30, 15, 5 ou 1 minute(s), de moins de 60, 45, 30, 15, 10 ou 1 seconde(s), (3), une irradiation, notamment à l'aide de rayons ionisants ;
(f), Un procédé de sélection magnétique, appelé ledit procédé de sélection magnétique, qui consiste à isoler la partie centrale des magnétosomes ou les magnétosomes traités de substances peu ou pas magnétiques, définies comme étant des débris bactériens, des bactéries non lysées, du fer dissout, des magnétosomes dissouts ou de toute matière contenue dans n'importe quelle suspension des séquences (i) ou (ii) qui contient peu ou pas de magnétosomes, préférentiellement moins de 10⁹, 10⁸, 10⁷, 10⁶, 10⁵, 10⁴, 10³, 10² ou 10 magnétosomes par millilitre de préparation. Pour cela, on peut appliquer un gradient de champ magnétique à n'importe quelle suspension des séquences (i) ou (ii), où une telle suspension est préférentiellement contenue dans un récipient tel qu'un tube ou une bouteille, préférentiellement en verre ou dans un matériau qui n'adsorbe pas ou peu les magnétosomes modifiés ou les parties centrales des magnétosomes. Ce gradient de champ magnétique permet notamment de faire migrer les substances magnétiques, préférentiellement les parties centrales des magnétosomes ou les magnétosomes modifiés, vers la zone où le champ magnétique est le plus intense tel que le paroi du récipient, de les concentrer dans cette zone et ainsi de les isoler. Ce gradient de champ magnétique est de préférence d'intensité supérieure à celle du champ magnétique terrestre, à 0,1, 1, 10 ou 100µT, 0,1, 1, 10 ou 100mT, 0,1, 1, 10, 50 ou 100T. Il est notamment d'intensité suffisante pour permettre de séparer des substances magnétiques telles que les parties centrales des magnétosomes ou les magnétosomes modifiés des substances peu ou pas magnétiques. Ce gradient de champ magnétique est de préférence d'intensité inférieure à 100 ou 50T, le plus préférentiellement à 5 ou 1T, 100, 50, 10 ou 1mT. Il peut notamment être d'intensité suffisamment faible pour éviter d'attirer les substances peu ou pas magnétiques. Un tel gradient de champ magnétique peut être appliqué à l'aide d'un aimant, tel qu'un aimant de Néodyme, un électro-aimant ou n'importe quel instrument ou matériau permettant de générer un champ magnétique dont l'intensité varie préférentiellement spatialement et/ou temporellement. Le gradient de champ magnétique peut être appliqué pendant plus d'1, 5, 10, 30, 45 ou 60 seconde(s), de 5, 10, 30, 45 ou 60 minutes, de 2, 5, 10, 15 ou 20 heures, d'1, 2 ou 5 jour(s), d'1, 2, 3 ou 4 semaine(s), de 2, 4 ou 6 mois. Suite à l'application du gradient de champ magnétique, le surnageant de la suspension qui ne contient pas ou qui contient en faible quantité les parties centrales des magnétosomes ou les magnétosomes modifiés peut être retiré et remplacé par une substance telle qu'un solvant, préférentiellement apyrogène, tel que l'eau apyrogène ;
(g), un autre procédé de sélection, appelé ledit autre procédé de sélection, durant lequel les parties centrales des magnétosomes ou les magnétosomes modifiés peuvent être isolés des débris bactériens, notamment à l'aide d'un système de filtration tangentielle possédant des colonnes avec des pores dont la taille permet soit de laisser traverser les débris bactériens, mais pas les parties centrales des magnétosomes soit de laisser traverser les parties centrales des magnétosomes, mais pas les débris bactériens. Cette taille est notamment inférieure à 100, 50, 20, 10, 5, 1, 0,5, 0,2, 0,1, 0,05, 0,02, 0,01, 0,005 ou 0,0001 µM. Cette taille peut être comprise entre préférentiellement 0,02 µm et 2 µm, préférentiellement entre 0,2 µm et 1 µm, le plus préférentiellement entre 0,2 µm et 0,4 µm ;

Dans un mode de réalisation, la séquence (i) est précédée d'une séquence de culture d'un organisme vivant, tel qu'une bactérie magnétotactique, qui synthétise des magnétosomes. Dans un mode de réalisation, la séquence de culture de l'organisme vivant se fait dans des conditions non totalement contrôlées, telles que les conditions de culture de bactéries magnétotactiques AMB-1 en bouteille.

Dans un autre mode de réalisation, la séquence de culture de l'organisme vivant se fait dans des conditions contrôlées, telles que les conditions de cultures de bactéries magnétotactiques MSR-1 en fermenteur qui font intervenir : (a), une solution acide nutritive contenant du fer pour garder le pH du milieu de culture constant durant la croissance des bactéries et (b), un apport d'air durant la croissance des bactéries pour favoriser cette croissance tout en gardant la concentration en oxygène du milieu de culture à une valeur faible, préférentiellement inférieure à 20 mbar, le plus préférentiellement inférieure à 2 mbar, pour permettre la synthèse des magnétosomes.

Dans un mode de réalisation, la séquence (i) est subdivisée en trois séquences réalisées préférentiellement mais pas obligatoirement dans l'ordre indiqué: (i1), lyse des bactéries magnétotactiques, (i2), retrait de la matière, notamment organique, qui ne fait pas partie de la partie centrale des magnétosomes et qui n'a pas pu être enlevée en il, (i3), récupération et lavage de la suspension contenant les parties centrales des magnétosomes. Toutes ces séquences peuvent être réalisées à l'aide de n'importe lequel ou n'importe quelle combinaison desdits procédés (a) à (g), répétée une ou plusieurs fois.

Dans un mode de réalisation, la séquence (i1) peut débuter par la concentration des bactéries magnétotactiques, notamment par centrifugation à 1000-10000g et/ou en utilisant ledit autre procédé de sélection, préférentiellement à l'aide d'un système de filtration tangentielle dans lequel la taille des trous est inférieure à la taille des bactéries magnétotactiques. La concentration en bactéries à la fin de cette séquence peut être entre 1,1 et 10⁴ fois supérieure à la concentration bactérienne mesurée au démarrage de (i1). Ensuite, les bactéries magnétotactiques, concentrées ou non, peuvent être lysées. La lyse peut être réalisée par choc osmotique, à l'aide dudit procédé chimique dans lequel ladite solution chimique est préférentiellement un tampon de lyse. Elle peut être réalisée en ajoutant un volume plus important de tampon de lyse que de volume de bactéries, préférentiellement égal à 2 à 100 fois le volume de lysat bactérien, le plus préférentiellement égal à 4 fois le volume de bactéries. A la fin de cette séquence, est obtenue une suspension de magnétosomes traités, extraits des bactéries, mélangés à des débris bactériens et possiblement à une certaine quantité de bactéries magnétotactiques non lysées.

Dans un autre mode de réalisation, la séquence (i2) consiste à traiter les suspensions de magnétosomes obtenus en fin de (i1) pour enlever les restes de matière, notamment organique, préférentiellement à l'aide de ladite solution chimique, préférentiellement à l'aide de détergents, tels que le phénol ou le dichlorométhane, permettant de retirer la membrane ou la matière, notamment organique, entourant les magnétosomes traités.

Dans un autre mode de réalisation, la séquence (i3) consiste à laver plusieurs fois la suspension obtenue en (i2) pour enlever tous les restes de détergents et pour obtenir une suspension contenant les parties centrales des magnétosomes.

Dans un mode de réalisation, la séquence (ii) consiste à couvrir la partie centrale des magnétosomes par un revêtement, préférentiellement apyrogène. Elle peut être réalisée à l'aide dudit procédé chimique de revêtement, notamment en mélangeant la suspension contenant les parties centrales des magnétosomes obtenue en fin de (i3) avec une suspension contenant le revêtement. Ce mélange peut être réalisé par homogénéisation, par sonication, préférentiellement une sonication de faible puissance, de moins de 500, 250, 100, 50, 25, 10, 5, 2 ou 1 Watt, notamment à l'aide d'un doigt soniquant ou au bain soniquant, par agitation, par chauffage, par rayonnement et ainsi permettre au revêtement d'adhérer ou de s'associer à la surface de la partie centrale des magnétosomes. Le mélange peut être effectué à pH neutre, acide ou basique, préférentiellement à un pH permettant de favoriser les interactions et/ou réactions chimiques résultant en une association entre le revêtement et la partie centrale des magnétosomes. La masse de revêtement utilisée est préférentiellement comprise entre un millième et mille fois la masse des parties centrales des magnétosomes, préférentiellement entre un centième et cent fois cette masse, le plus préférentiellement entre un dixième et dix fois cette masse. A la fin de la séquence (ii) est obtenue une suspension contenant les parties centrales des magnétosomes couvertes d'un revêtement apyrogène.

Dans un mode de réalisation, la partie centrale des magnétosomes obtenue en fin de séquence (i3) est stérilisée, en utilisant préférentiellement une méthode qui ne dénature pas cette partie centrale telle que l'autoclavage. La séquence (ii) est alors préférentiellement réalisée dans un environnement stérile afin d'obtenir une préparation apyrogène à l'issue de la séquence (ii). Dans un mode de réalisation, la suspension obtenue en fin de séquence (ii) est stérilisée, préférentiellement en utilisant une méthode telle que les rayons gamma qui ne détruit pas le revêtement et la partie centrale des magnétosomes.

Dans un mode de réalisation, le rendement d'obtention de la suspension contenant les parties centrales des magnétosomes revêtues est estimé comme étant égal à la quantité de fer contenue dans cette suspension divisée par la quantité de fer initiale contenue dans la suspension de bactéries magnétotactiques avant la séquence de lyse. Ce rendement est préférentiellement supérieur à 1, 2, 5, 10, 25, 50, 75 ou 100%. Ce rendement peut être optimisé en combinant plusieurs desdits procédés les uns avec les autres et/ou en répétant plusieurs fois l'un ou plusieurs du(des)dit(s) procédé(s).

### Exemples expérimentaux :

### Description des tableaux:

**Tableaux 1 et 2:** Propriétés de différents types de suspensions, contenant des BNF-Starch, des bactéries entières, des parties centrales des magnétosomes non revêtues, des parties centrales des magnétosomes revêtues de poly-L-lysine, chitosan, carboxy-methyldextran, acide citrique, acide oléique, silice, acide folique, DOPC, alendronate, néridronate, PEI, Al(OH)₃ où les espèces de bactéries magnétotactiques sont AMB-1 et MSR-1. **Dans le tableau 1** : concentration en endotoxines mesurée en UE par milligramme de fer par millilitre de suspension, pourcentage de diminution au bout de 20 minutes de l'absorption, mesurée à 480 nm, d'1mg des différentes suspensions, variation de la température (ΔT) et pente à l'origine de cette variation (δT/δt) pour les suspensions mises en présence de cellules GL-261 traitées suivant la condition 3, pourcentage de cellules vivantes pour les suspensions mises en présences de cellules GL-261 traitées suivant les conditions 1, sans champ (-B), et suivant la condition 2, avec champ avec une élévation de température maximum de 45°C, %cellules vivantes (+B). Le pourcentage de cellules vivantes est le nombre de cellules vivantes traitées suivant les conditions 1 ou 2 divisé par le nombre cellules vivantes non traitée à 37°C. Pour la première étude avec les BNF-Starch, le pourcentage de cellules vivantes est le nombre de cellules vivantes traitées suivant les conditions 1 ou 2 divisé par le nombre cellules vivantes non traitées à 45°C. **Dans le tableau 2** : épaisseur du revêtement en nm, point isoélectrique en unité de pH, taille hydrodynamique en nm, potentiel zéta mesuré en mV en fonction du pH, des nanoparticules synthétiques, revêtues on non, contenues dans les différentes suspensions, pourcentage en azote (%N), en carbone (%C), en hydrogène (%H), en soufre (%S) dans les nanoparticules synthétiques, revêtues ou non.

**Tableau 3:** Propriétés de liaison des parties centrales entre elles pour différents revêtements.

### EXEMPLES:

### Matériels et méthodes:

**Détermination de la concentration en fer des différentes suspensions de nanoparticules :** Les nanoparticules sont d'abord dissoutes par de l'acide chlorhydrique 12N et les ions Fe²⁺ sont oxydées en ions Fe³⁺ avec de l'eau oxygénée. Du thiocyanate de potassium est ensuite utilisé pour complexer les ions Fe³⁺ et la concentration en Fe³⁺ est déterminée par mesure d'absorption du complexe à 476 nm.

**Microscopie à transmission électronique (MET) :** Le MET est utilisé pour déterminer la taille, la distribution en taille des différentes nanoparticules, l'épaisseur du revêtement des nanoparticules et le type d'organisation des nanoparticules. Pour réaliser les études en MET, les différentes suspensions sont lavées à deux reprises et resuspendues dans de l'eau MilliQ autoclavée pour obtenir une concentration en fer de 300 µg/ml. 5 µl de chaque suspension sont déposés sur des grilles de carbone. Les grilles sont séchées pendant au moins 2 heures à température ambiante pour être ensuite observées au MET (JEOL LaB6 JEM-2100).

**Test de Lysat d'Amébocytes de Limules (LAL) :** Les tests LAL sont réalisés en conditions stériles à l'aide du kit 88282 de ThermoScientific appelé "Pierce LAL Chromogenic Endotoxin Quantitation Kit". 1 ml de chaque suspension, homogénéisée par sonication et lavée avec de l'eau apyrogène, est d'abord chauffé à 70°C pendant 10 minutes au bain sec pour dénaturer d'éventuelles protéines résiduelles qui pourraient fausser les résultats du test LAL. 25 µl de chaque suspension contenant 10 µg de fer sont ensuite introduits dans les puits d'une microplaque maintenue à une température de 37°C pendant toute la durée de l'expérience. 25 µl de la solution du kit LAL sont ajoutés pour initier la réaction. Après 10 minutes de réaction, 50 µl du substrat chromogénic sont introduits dans les puits pendant 6 minutes pour permettre la détection de la quantité d'endotoxines. Enfin 25 µl d'acide acétique sont ajoutés pour stopper la réaction. La densité optique des suspensions obtenues est mesurée à 405 nm à l'aide d'un lecteur de microplaque. La concentration en endotoxines est alors estimée à l'aide d'une gamme étalon fournie avec le kit. Afin de vérifier que le test LAL n'interfère pas avec les nanoparticules, un taux de recouvrement, défini comme étant égal à Cₜₒₜₐₗ/C₁+C₂ est mesuré, où Cₜₒₜₐₗ est la concentration en endotoxines des suspensions de nanoparticules mélangées à une quantité connue d'endotoxines de 0,5 UE/mL, C₁ est la concentration en endotoxines dans les différentes suspensions de nanoparticules et C₂ = 0,5 EU/mL. Le taux de recouvrement estimé lors des différentes mesures est supérieur à 50 %, ce qui indique que les nanoparticules n'interfèrent pas avec le test LAL.

**Analyseur élémentaire de carbone, hydrogène, azote et soufre (CHNS):** Des mesures sont réalisées à l'aide d'un analyseur CHNS (Analyseur Elémentaire Flash EA 1112 de chez Thermo Fischer scientific) à partir de 10 mg en fer de chaque suspension, lyophilisés, permettant de déterminer les pourcentages en carbone, azote, hydrogène et soufre de ces suspensions. **Mesures de diffusion :** Le potentiel zéta et la taille hydrodynamique (diamètre hydrodynamique dans le cas d'objets sphériques) des différentes nanoparticules sont mesurés à l'aide du Zetasizer Nano ZS de Malvern Instruments. On identifie les objets sphériques à la forme exponentielle décroissante de la fonction de corrélation. Pour les mesures, les suspensions de nanoparticules contiennent 30 µg/ml de fer et sont à un pH ajusté entre 2 et 12 à l'aide de solutions d'acide chlorhydrique et d'hydroxyde de sodium.

**Mesures d'absorption :** La variation au cours du temps de l'absorbance des différentes suspensions de nanoparticules est mesurée à 480 nm à l'aide d'un spectrophotomètre d'absorption UviLine9400 Secomam.

**Détermination de la concentration en amine primaire pour 1 mg en fer de la suspension de partie centrale de la nanoparticule synthétique:** Une méthode pour contrôler la pureté de la partie centrale est le dosage des amines via le TNBSA (acide 2, 4, 6-trinitrobenzène sulfonique), qui a la particularité de réagir sur des amines primaires. On commence par ajouter à 1 mg de fer de la partie centrale dans une solution tampon à 0.1 M de bicarbonate de soude à pH 8.5 puis on ajoute du TNBSA (R'-SO₃H), ce qui conduit à la réaction suivante : R - NH₂ + R'- SO₃H <=> H₂SO₃ + R' - NH - R. Cette réaction se fait à 37°C pendant 2h et le composé obtenu étant coloré en jaune (R' - NH - R), sa concentration en amine est déterminée par absorption à 405 nm. Cette concentration est équivalente à celle de la partie centrale. Une courbe d'étalonnage est réalisée avec de la glycine qui possède une fonction amine primaire.

**Détermination de la concentration en phosphate pour 1 mg de fer de la suspension de partie centrale de la nanoparticule synthétique :** Le dosage des groupements phosphates de la partie centrale est déterminée par colorimétrie. On fait d'abord réagir du molybdate d'ammonium ((NH₄)₂MoO₄), en présence de phosphate (issus d'une solution mère (DOPC) pour l'étalonnage ou provenant des échantillons à doser après digestion avec de l'acide perchlorique à 70 % pendant 2 h à 130°C). Le précipité de Molybdate est jaune et instable. On y ajoute très rapidement de l'acide ascorbique qui va réduire ce complexe pour donner un sel de molybdate de couleur bleue qui est un composé stable (on chauffe pendant 5 min à 100°C au bain sec pour activer cette réduction) puis on mesure la quantité de phosphate complexée par absorbance à 800 nm.

**Toxicité cellulaire et mesure de température des différentes nanoparticules soumises ou non l'application d'un champ magnétique alternatif** : Les cellules GL261 sont ensemencées dans une flasque T175 jusqu'à atteindre 70-80 % de confluence, le surnageant est retiré, 4 ml de trypsine-EDTA à 0,25% sont ajoutés aux cellules, les cellules sont incubées pendant 5 minutes, puis sont décollées. La trypsine est désactivée en ajoutant 30 ml de milieu cellulaire. Les cellules sont ensuite diluées à une concentration de 1,25 10⁺⁶ cellules par millilitre après centrifugation à 700 tours par minute pendant 10 minutes à 4°C. 400µl de cellules sont introduits dans des tubes eppendorf afin d'atteindre ∼ 5.10⁺⁵ cellules par condition. On ajoute dans les tubes les différentes suspensions de nanoparticules synthétiques à une concentration finale de 1 mg/ml en fer. Les tubes eppendorf sont ensuite chauffés pendant 10 minutes à 37°C. S'ensuit une série de 3 conditions. Pour la condition 1 de traitement, les tubes sont maintenus à 37°C pendant 30 minutes à l'aide d'un bain chauffant à sec. Pour la condition 2 de traitement, les tubes sont maintenus à 45°C pendant 30 minutes par application d'un champ magnétique alternatif de fréquence 198 kHz et d'intensité moyenne ajustée entre 23 et 46 mT pour maintenir la température à 45°C. Pour la condition 3 de traitement, les tubes sont soumis à l'application d'un champ magnétique alternatif de fréquence 198 kHz et d'intensité moyenne 32 mT pendant 30 minutes. Les variations de température au cours du temps sont mesurées à l'aide d'une sonde thermocouple placée dans les tubes eppendorf. Après les traitements, le contenu des tubes est introduit dans une flasque T 25 auquel on ajoute 6 ml de milieu RPMI et 10% de sérum de veau fœtal. Les cellules sont incubées pendant 24 heures en présence de 5% de CO₂. 24 heures après, un test de viabilité cellulaire est effectué au bleu de Trypan, permettant de discriminer les cellules vivantes incolores des cellules mortes colorées.

### Exemple 1: Caractérisation des suspensions contenant des BNF-Starch.

Des nanoparticules synthétisées chimiquement par la société Micromod, appelés BNF-Starch (Référence : 10-00-102), sont testées. Ces nanoparticules d'oxyde de fer sont entourées d'hydroxyéthylamidon et ont un diamètre hydrodynamique de 119 nm. Elles ont un point isoélectrique de pH 9,5, un potentiel zéta qui varie de 7 mV à pH 2 à -20 mV à pH 12 et un pourcentage de carbone mesuré au CHNS de 8,7%. Les mesures MET ont permis d'estimer l'épaisseur du revêtement, de 1 à 4 nm. La variation d'absorption au cours du temps, mesurée à 480 nm, d'une suspension contenant 1 mg de BNF-Starch ne diminue pas en 20 minutes, indiquant la stabilité de cette suspension. Un test LAL, réalisé sur ces suspensions, révèle un faible taux d'endotoxines (< 50 EU/mg/ml). Pour une première série de mesures, le tableau 1 montre que lorsqu'un mélange d'une suspension de BNF-Starch et des cellules GL-261 est soumis à la condition 3 du traitement, la température du mélange augmente faiblement de 6,2 °C en passant de 36,5°C avant application du champ à 42,7°C après 30 minutes d'application du champ. La pente à l'origine de la variation de température est estimée à 0,009 °C/sec. Lorsque ce même mélange est soumis à la condition 2 de traitement, le tableau 1 montre que le pourcentage de cellules vivantes est de 78±5%, similaire à celui de 71±5% obtenu lors de la condition 1 de traitement, sans champ. Cela indique la faible cytotoxicité induite par les BNF-Starch sur les cellules GL261 en présence du traitement selon la condition 2 par rapport à des cellules chauffées à 45°C. Lors d'une deuxième série de mesure, lorsque le mélange est soumis à la condition 2 de traitement, le tableau 1 montre que le pourcentage de cellules vivantes est de 31%, plus faible que 86% obtenu lors de la condition 1 de traitement, sans champ lorsque ce pourcentage est estimé par rapport au nombre de cellules vivantes à 37 °C. Cela indique la cytotoxicité induite par les BNF-Starch sur les cellules GL261 en présence du traitement selon la condition 2 par rapport à des cellules chauffées à 37 °C.

### Exemple 2: Chaînes de magnétosomes pyrogènes extraites de la souche AMB-1.

**Préparation:** Des bactéries *Magnetospirillum* AMB-1 (ATCC, souche 79024) sont d'abord introduites dans du milieu de culture stérile contenant les nutriments et additifs nécessaires à la prolifération des bactéries magnétotactiques et à la production des magnétosomes (milieu ATCC 1653) et les milieux sont ensuite placés dans un incubateur à 30°C pendant 7 jours. Au bout de 7 jours, les milieux sont centrifugés, le culot bactérien est lavé, les bactéries magnétotactiques sont concentrées à l'aide d'un aimant, introduites dans un tube contenant 1mL de TRIS 0.05M, soniquées à l'aide d'un doigt soniquant pendant 2 heures à 30W à 0°C puis lavées 17 fois à l'eau Millipore® stérile à l'aide d'un aimant. Est obtenue une suspension contenant des chaînes de magnétosomes pyrogènes extraites des bactéries magnétotactiques. **Caractérisation:** Les mesures MET ont mis en évidence la présence dans ces suspensions de chaînes de magnétosomes avec des longueurs de chaînes comprises entre 50 et 800 nm, des tailles de magnétosomes comprises entre 5 nm et 60 nm. L'épaisseur du revêtement est de 1 à 5 nm. L'absorption de ces suspensions contenant 1 mg de fer, mesurée à 480 nm, diminue de 30% au bout de 20 minutes, ce qui montre la faible sédimentation de ces suspensions. Des mesures de diffusion de lumière effectuées sur ces chaînes indiquent la présence de trois populations de chaînes, 5% de taille hydrodynamique (TH) 176 nm, 81% de TH 986 nm et 14% de TH 4363 nm. Elles ont un point isoélectrique de pH 4,2, un potentiel zéta qui varie de 20 mV à pH 2 à -38 mV à pH 12. L'analyse au CHNS révèle un pourcentage de carbone dans ces chaînes de 13,9%. La concentration en endotoxines de ces suspensions, mesurée par le test LAL, a été estimée à une valeur élevée comprise entre 18 000 et 150 000 UE par ml par mg d'oxyde de fer. La présence d'endotoxines a également été suggérée par des mesures d'absorption infra-rouge à transformée de Fourrier à l'aide d'un spectromètre Nicolet FT-IR modèle 380. Ces spectres indiquent en effet la présence de pics à 1250 cm⁻¹ et 1050 cm⁻¹, pouvant être attribués aux vibrations des groupements phosphates des lipopolysaccharides et des phospholipides. Lors d'une première série de mesures, lorsque les chaînes de magnétosomes pyrogènes extraites d'AMB-1 sont mélangées à des cellules GL-261 et soumises et que le mélange est soumis à la condition 3 de traitement, la température du mélange augmente de 20,5°C, de 36°C avant application du champ à 56.5°C après 30 minutes d'application du champ. La pente à l'origine de la variation de température est estimée à 0.043°C/sec. Cette augmentation de température est plus importante que celle observée pour les BNF-Starch. Lors d'une deuxième série de mesures, lorsque ce mélange est soumis à la condition 2 de traitement en présence du champ, le tableau 1 montre que le pourcentage de cellules vivantes est faible à 10% et inférieur à 55% obtenu lors de la condition 1 de traitement, sans champ. Cela indique la cytotoxicité induite par ces magnétosomes pyrogènes sur les cellules GL261 en présence du traitement selon les conditions 1 et 2 avec une plus grande cytotoxicité pour la condition 2 (en présence du champ) que pour la condition 1 (en absence du champ).

**Efficacité anti-tumorale sur des tumeurs U87-Luc implantées dans le cerveau de souris:** Les expériences d'efficacités sont réalisées sur 7 groupes de dix souris chez lesquelles on fait pousser des tumeurs intracérébrales U87-Luc ayant des volumes compris entre 1 et 29 mm³. Durant ces expériences, les souris sont alimentées selon les procédures en vigueur et abreuvées à volonté. L'état général des animaux est suivi quotidiennement, les souris sont pesées tous les deux jours et sont euthanasiées lorsqu'une diminution de leur poids corporel supérieur à 15%, des signes de douleur, une posture inhabituelle, sont observés. Les 7 différents groupes de souris reçoivent 8 jours après l'injection des cellules U87-Luc au point d'injection des cellules tumorales (2.2.0): une solution de 2 µL de NaCl à 0,9 % (groupes 1 et 2) ; 2 µL d'une suspension de magnétosomes à 20 mg/mL en maghémite (groupes 3, 4 et 5) ; 2 µL d'une suspension de BNF-Starch à 20 mg/mL en maghémite (groupes 6 et 7). 8 jours après injection des cellules U87-Luc, les volumes tumoraux moyens pour les groupes 1, 2, 3, 4, 5, 6 et 7 sont respectivement de 29, 10, 5, 3, 25, 10 et 2 mm³. Les groupes de souris 2, 4, 5 et 7 sont exposés 3 fois par semaine pendant 5 semaines à un champ magnétique alternatif d'intensité moyenne 25 mT et de fréquence 198 kHz pendant 30 minutes. Des études histologiques sont réalisées sur les souris du groupe 4 pour déterminer si la tumeur disparait complètement 150 jours après l'administration des chaînes de magnétosomes. Les tissus étudiés en histologie sont prélevés de souris euthanasiées, les cerveaux sont extraits, fixés avec une solution à 4 % de paraformaldéhyde, découpés en tranches transversales de 2 mm d'épaisseurs, inclus dans des blocs de paraffine de 3 µm d'épaisseur, recueillis sur des lames de verre puis colorés avec de l'hématoxyline-éosine (H&E) pour distinguer la zone saine de la zone tumorale. Enfin, la température est mesurée pendant les différents traitements à l'aide d'une caméra infra-rouge. Chez les souris contenant uniquement des tumeurs, traitées par la seule administration de nanoparticules (chaîne de magnétosomes et BNF) ou par les seules applications multiples du champ magnétique alternatif, les volumes tumoraux augmentent rapidement pour atteindre un volume moyen de 150 mm³ en moins de 40 jours suivant l'administration des cellules tumorales. Les temps de survie moyen des souris des groupes 1, 2 et 6 ont été estimés à 38 jours en moyenne. Ces résultats suggèrent que ni la seule application du champ magnétique alternatif, ni la seule administration des BNF-Starch ou des chaînes de magnétosomes n'a eu d'effet anti-tumoral significatif sur les tumeurs U87-Luc.

Chez les souris traitées par administration de chaînes de magnétosomes et applications multiples du champ magnétique, une légère augmentation de température est observée pendant les trois premières séances d'application du champ, qui est similaire pour les souris des groupes 4 et 5 et qui est de 4°C (première séance), 2°C (deuxième séance) et 0.5°C (troisième séance). Aucune augmentation de température n'est observée lors des séances suivantes d'application du champ des souris des groupes 4 et 5. Pour tous les autres groupes, aucune augmentation de température n'est observée. Pour les souris du groupe 5 possédant des tumeurs de grande taille (∼ 25 mm³) une diminution significative du volume tumorale durant les 7 jours qui suivent l'administration des chaînes de magnétosomes est observée, de 64%. Globalement, le volume tumoral augmente de manière beaucoup moins prononcée pour le groupe 5 que pour les groupes 1, 2, 3 et 6 durant les 28 jours qui suivent l'administration des chaînes de magnétosomes. Par ailleurs, les souris du groupe 5 vivent en moyenne une semaine de plus que les souris des groupes 1, 2 et 6. Pour 40% des souris du groupe 4 possédant de petites tumeurs (∼ 3 mm³) le volume tumoral moyen décroit en 51 jours suivant l'administration des chaînes de magnétosomes jusqu'à la disparition totale de la tumeur. Ces souris sont totalement guéries. En effet, ces souris sont encore en vie 143 jours après l'administration des chaînes de magnétosomes (J143). La guérison totale de ces souris a été confirmée par l'étude de coupes histologiques de leurs cerveaux, prélevés à J143, montrant l'absence de tumeurs et lésions. Pour les souris du groupe 7, traitées à l'aide des BNF-Starch, la croissance du volume tumorale moyen et le temps de 45 jours sont similaires à ceux des souris des groupes 1, 2 et 6. Aucun effet anti tumoral n'est observé.

Nous pouvons conclure que : (i), pour 40% de souris avec un volume moyen de tumeurs U87-Luc traitée de 3mm³, il est possible de détruire entièrement ces tumeurs en administrant 40 µg en maghémite d'une suspension de chaînes de magnétosomes pyrogènes extraites d'AMB-1 dans ces tumeurs et en exposant ces tumeurs à de multiples applications d'un champ magnétique alternatif d'intensité moyenne 25 mT et de fréquence 198 kHz, (ii), l'activité anti-tumorale observée avec les chaînes de magnétosomes est importante alors qu'aucune activité anti- tumorale n'est observée avec les BNF-Starch.

### Exemple 3: Chaînes de magnétosomes pyrogènes extraites de la souche MSR-1.

**Préparation:** Des bactéries MSR-1 sont d'abord cultivées à 30°C pendant 5 à 7 jours sur un gel d'agar en présence de fer et d'une faible concentration en oxygène (∼0.5% O₂). Des colonies magnétiques sont prélevées et cultivées à 30°C en présence d'air pendant plusieurs jours dans un milieu de préculture sans fer contenant des sources de carbone, d'azote, de minéraux, d'éléments trace et d'extraits de levure. Les bactéries magnétotactiques issues de la préculture sont cultivées dans un fermenteur de 50 litres à 30°C dans un milieu similaire au milieu de préculture. Pendant la croissance, le pH est maintenu à 6,8-7 par ajout d'un milieu nutritif acide contenant une source de fer et de l'air comprimé est introduit dans le milieu de culture pour favoriser la croissance bactérienne tout en gardant la concentration en oxygène inférieure à 0,2% pour permettre la synthèse des magnétosomes. Les bactéries MSR-1 issues de la fermentation sont concentrées jusqu'à une densité optique, mesurée à 565 nm (DO₅₆₅ₙₘ), de 110-120. 100 mL de ce concentrât bactérien sont ensuite mélangés à 400 mL de NaOH 5M et chauffés à 60°C pendant 1h30 à 2h00 pour lyser les bactéries. Les magnétosomes traités sont ensuite isolés des débris bactériens en plaçant un aimant de Néodinium pendant une nuit contre la paroi du récipient contenant la suspension de bactéries lysées et en remplaçant le surnageant contenant la soude et les débris bactériens par du PBS 1X. La suspension obtenue est ensuite soniquée pendant 20 secondes à 10W en présence de PBS 1 X, placée contre un aimant de Néodinium pendant 15 minutes, le surnageant est retiré et les magnétosomes traités sont resuspendus dans du PBS 1X. Cette séquence de sonication et de séparation magnétique est répétée quatre fois. Des chaînes de magnétosomes pyrogènes extraites de la souche MSR-1 sont ainsi obtenues.

**Caractérisations:** Les mesures MET ont mis en évidence la présence dans ces suspensions de chaînes de magnétosomes avec des longueurs de chaînes comprises entre 200 et 1500 nm, des tailles de magnétosomes comprises entre 20 nm et 60 nm. L'absorption des suspensions contenant 1 mg en fer de ces chaînes, mesurée à 480 nm, diminue de 86% au bout de 20 minutes, ce qui montre la faible stabilité de ces suspensions. Des mesures de diffusion de lumière effectuées sur ces chaînes indiquent la présence de deux populations de chaînes de tailles hydrodynamiques 535 nm et 2822 nm. Elles ont un point isoélectrique de pH 6,4, un potentiel zéta qui diminue de 15 mV à pH 2 à-31 mV à pH 12. L'analyse au CHNS révèle un pourcentage de carbone dans ces chaînes de 4,1% lors de la première mesure et de 12,2% en moyenne sur les 9 mesures suivantes. Lors d'une deuxième série de mesures, la concentration en endotoxines de ces suspensions, mesurée par le test LAL, a été estimée à une valeur élevée comprise entre 2000 et 17 000 UE par ml par mg de fer. La présence d'endotoxines a également été suggérée par des mesures d'absorption infra-rouge à transformée de Fourrier à l'aide d'un spectromètre Nicolet FT-IR modèle 380. Ces spectres indiquent en effet la présence de pics à 1150 cm⁻¹ et 1030 cm⁻¹, pouvant être attribués aux vibrations des groupements phosphates des lipopolysaccharides et des phospholipides. Lors d'une première série de mesure, lorsque les chaînes de magnétosomes pyrogènes extraites de MSR-1 sont mélangées à des cellules GL-261 et soumises à la condition 3 de traitement, la température du mélange augmente de 9,4°C, de 36,2°C avant application du champ à 45,6°C après 30 minutes d'application du champ. La pente à l'origine de la variation de température est estimée à 0,012°C/sec lors de la première mesure et à 0,019°C/sec lors de la seconde mesure. Cette augmentation de température est plus importante que celle observée pour les BNF-Starch. Lorsque ce mélange est soumis à la condition 2 de traitement, le tableau 1 montre que le pourcentage de cellules vivantes est faible à 5% lors de le première mesure et 12% lors de la seconde et est inférieur à 39% obtenu lors de la condition 1 de traitement lors de la première et seconde mesure, sans champ. Cela indique la cytotoxicité induite par ces magnétosomes pyrogènes sur les cellules GL261 en présence du traitement selon les conditions 1 et 2 avec une plus grande cytotoxicité pour la condition 2 (en présence du champ) que pour la condition 1 (en absence du champ).

### Exemple 4: Parties centrales des magnétosomes issues de MSR-1.

**Préparation:** 100 µl de la suspension contenant des chaînes de magnétosomes pyrogènes extraites de la souche MSR-1 obtenues dans l'exemple 3 sont mélangés à 200 mL d'une solution contenant du Triton X-100 à 1 % et du SDS à 1%, le mélange est chauffé pendant une nuit à 50°C, est placé contre un aimant de Néodinium, le surnageant est retiré et remplacé par 80 mL de phénol à pH 8. La suspension obtenue est chauffée pendant 2 heures en soniquant à 60°C, maintenue pendant une nuit à 60°C sans sonication, placée contre un aimant, le surnageant de la suspension est retiré et remplacé par 80 mL de chloroforme. La suspension contenant le chloroforme est placée contre un aimant de Néodinium, le surnageant est retiré et le chloroforme résiduel adsorbé à la surface des magnétosomes traités est enlevé en chauffant ces magnétosomes pendant 2 heures sous hotte. Enfin les parties centrales des magnétosomes obtenues sont désorbées de la paroi en verre des tubes qui les contiennent en ajoutant 80 mL de NaOH à 1M chauffés pendant 1 heure à 60°C au bain soniquant. La suspension contenant les parties centrales des magnétosomes est placée contre un aimant de Néodinium, le surnageant est retiré et remplacé par de l'eau MilliQ stérile, la suspension est soniquée pendant 20 secondes à 10 W. Cette séquence de lavage est répétée quatre fois. La suspension contenant les parties centrales des magnétosomes est dégazée à l'azote pour éviter l'oxydation, stérilisée par autoclavage et stockée à -80°C.

**Caractérisations:** Les mesures MET révèlent l'absence de revêtement autour des parties centrales des magnétosomes. L'absorption des suspensions contenant 1 mg de la partie centrale des magnétosomes, mesurée à 480 nm, diminue de 60 à 80% au bout de 20 minutes, ce qui montre la faible stabilité de ces suspensions. La concentration en endotoxines de ces suspensions contenant a été estimée à une valeur comprise entre 10 et 100 UE par millilitre par mg d'oxyde de fer, ce qui montre la nature apyrogène de cette suspension. Le point isoélectrique de ces suspensions a été mesuré à un pH acide de 3,5. Par ailleurs, entre pH 4 et pH 6, on observe une augmentation du potentiel zéta de -8 mV à -1.5 mV puis entre pH 6 et pH 8 une diminution du potentiel zéta de -1.5 mV à -27 mV, ce qui semble être caractéristique de la présence d'agrégats. Les mesures de diffusion de lumière à l'aide du zétasizer révèlent la présence de 79% d'agrégats de taille sphérique hydrodynamique 3076 nm et 21% d'agrégats de taille sphérique hydrodynamique sphérique 677 nm. L'analyse au CHNS de ces suspensions révèle notamment des concentrations en carbone de 3,3%, en azote de 0,2%. Ces concentrations sont plus faibles que celles mesurées pour les chaînes de magnétosomes pyrogènes extraites de MSR-1 (%N=0,7 et %C=4,1) et pour les bactéries entières MSR-1 lyophilisées (%N=11 et %C=49). Ces résultats suggèrent que la quantité de matière organique entourant la partie centrale des magnétosomes est nettement plus faible dans l'échantillon contenant les parties centrales des magnétosomes que dans ceux contenant les bactéries entières MSR-1 ou les chaînes de magnétosomes pyrogènes extraites de MSR-1. Le dosage amine nous indique qu'il y a entre 260 ng et 1,2 µg de fonctions amines présentes dans ou à la surface des parties centrales dans la suspension contenant 1 mg en fer de parties centrales. Le dosage des phosphates nous indique qu'il y a 10 µg de fonction phosphate présentes dans ou à la surface des parties centrales dans la suspension contenant 1 mg en fer de parties centrales. Lors d'une deuxième série de mesures, lorsque les parties centrales des magnétosomes sont mélangées à des cellules GL-261 et soumises à la condition 3 de traitement, la température du mélange augmente de 9,8°C après 30 minutes d'application du champ. La pente à l'origine de la variation de température est estimée à 0,012°C/sec. Lorsque ce mélange est soumis à la condition 2 de traitement, le tableau 1 montre que le pourcentage de cellules vivantes est faible à 0-9% et inférieur à 77% obtenu lors de la condition 1 de traitement, sans champ.

### Exemple 5: Parties centrales des magnétosomes issues d'AMB-1.

**Préparation:** La culture des bactéries magnétotactiques AMB-1 est réalisée selon le procédé décrit dans l'exemple 3. Au bout de 7 jours de culture, les bactéries sont concentrées à 25°C à l'aide de la filtration tangentielle (colonne 500kDa, 800tr/min) dans un volume de 1 litre. Elles sont ensuite mélangées sous agitation à une solution contenant 1mM d'EDTA et 30 µg/mL de protamine à pH=7,4, le milieu liquide est séparé des bactéries par filtration tangentielle, puis les bactéries obtenues sont mélangées avec une solution de PBS à pH=7,4, le milieu liquide est à nouveau séparé des bactéries par filtration tangentielle. La suspension de bactéries obtenue est ensuite diluée dans du PBS jusqu'à atteindre une densité optique, mesurée à 565 nm, de 5 permettant de lyser les bactéries. La suspension obtenue est soniquée pendant 30 minutes à 70 W à 0°C à l'aide d'une sonde de sonication en titane ayant un diamètre de 13 mm. La suspension ainsi obtenue est lavée une première fois en plaçant un aimant contre la paroi du récipient contenant la suspension, en retirant le surnageant, en le remplaçant par une solution contenant 10 mM d'HEPES et 200 mM de NaCl à 6°C et en soniquant par série de 3 pulses à 30W pendant 2 secondes. Elle est lavée 4 fois supplémentaires en utilisant une méthode similaire, en remplaçant le mélange d'HEPES et de NaCl par de l'eau apyrogène stérile. A la fin des lavages, sont obtenues des suspensions contenant des chaînes magnétosomes, comme cela a été vérifié en MET. A l'aide d'un aimant, les chaînes de magnétosomes sont isolées du surnageant, le surnageant est remplacé par un volume de 30 mL de TRI REAGENT (Sigma, référence : T9424), le tout est soniqué pendant 2 heures à 50°C avec un bain soniquant chauffant. La suspension obtenue est ensuite placée contré un aimant à 4°C pendant 1 heure, le surnageant est retiré et remplacé par une solution d'éthanoate de sodium et le mélange est soniqué pendant 30 minutes à 37°C avec un bain soniquant chauffant. Les magnétosomes traités sont ensuite lavés une première fois en isolant à l'aide d'un aimant le surnageant, en retirant le surnageant, en le remplaçant par une solution d'éthanoate de sodium puis en soniquant la suspension pendant 30 min à 37°C à l'aide d'un bain soniquant chauffant. Cette étape de lavage est répétée 3 fois supplémentaires de la même manière. Notamment pour éliminer la bicouche lipidique, la suspension est placée contre un aimant, le surnageant est retiré et remplacé par une solution contenant 1% de Triton X114, 1% de deoxycholate et 4mM EDTA. Ensuite, la suspension est mise sous agitation mécanique pendant 1 heure à 4°C, est placée contre un aimant, le surnageant est retiré et remplacé par la solution contenant 1% de Triton X114, 1% de deoxycholate et 4mM EDTA. Le mélange est mis sous agitation mécanique une heure mais à 37°C. La suspension est ensuite lavée une première fois en plaçant un aimant contre la paroi du récipient contenant la suspension, en retirant le surnageant, en le remplaçant par un mélange méthanol/tampon phosphate (v/v, 1/1). Ensuite, la suspension obtenue est lavée en étant placée contre un aimant, en retirant le surnageant, en le remplaçant par un mélange contenant du méthanol et un tampon phosphate puis en soniquant la suspension obtenue pendant 5 minutes à 30W à l'aide d'un doigt soniquant. Un second lavage est effectué de la même manière. La suspension est à nouveau lavée en plaçant la suspension contre un aimant, en retirant le surnageant, en le remplaçant par de l'eau stérile apyrogène et en soniquant avec un bain soniquant à 37°C pendant 1 heure. 5 lavages supplémentaires à l'eau sont réalisés de la même manière. La suspension obtenue est ensuite stérilisée par autoclavage à 121°C pour obtenir des suspensions contenant les parties centrales des magnétosomes issues d'AMB-1.

**Caractérisation:** Le MET révèle l'absence de revêtement autour des partie centrales des magnétosomes. L'analyse au CHNS de ces suspensions révèle un pourcentage en carbone de 4,9% qui est nettement plus faible que celui de la bactérie entière qui est 32% et des chaînes de magnétosomes pyrogènes (13,9%). Le taux d'endotoxines dans ces parties centrales est de 20 à 100 UE/mg/mL. L'absorption des suspensions contenant 1 mg de la partie centrale des magnétosomes, mesurée à 480 nm, diminue de 80 à 90% au bout de 20 minutes, ce qui montre la faible stabilité de ces suspensions. Le potentiel zéta de ces suspensions diminue globalement de 38 mV à pH 2 à - 60 mV à pH 12. Le point isoélectrique de ces suspensions a été mesuré à un pH acide de 4,9. Lors d'une deuxième série de mesure, lorsque les parties centrales des magnétosomes sont mélangées à des cellules GL-261 et soumises à la condition 3 de traitement, la température du mélange augmente de 23,8°C après 30 minutes d'application du champ. La pente à l'origine de la variation de température est estimée à 0,024°C/sec. Lorsque ce mélange est soumis aux conditions 2 et 1 de traitement, le pourcentage de cellules vivantes est de 48% et de 30% respectivement.

### Exemple 6: Parties centrales de magnétosomes issues de MSR-1, revêtues de poly-L-lysine.

**Préparation:** Les parties centrales des magnétosomes issus de MSR-1 décrits dans l'exemple 4 sont revêtues de poly-L-lysine sous hotte à flux laminaire dans des conditions stériles. La solution stocke de poly(L-lysine hydrobromide) de masse molaire 21000 g/mol (Gmac, CAS : 25988-63-0) contient 40 mg/mL de poly(L-lysine hydrobromide) préparée dans de l'eau apyrogène et filtrée avec un filtre PES (polyéthersulfone) de 0,45 µm, conservée à -80°C. Lors de la séquence de revêtement, la masse de poly-L-lysine utilisée est sept fois plus importante que la masse des parties centrales des magnétosomes. 25 mL d'une suspension contenant les parties centrales des magnétosomes à une concentration en fer de 3 mg/mL sont introduits dans un tube en verre, le tube est positionné contre un aimant de NdFeB de 1,3 T, le surnageant est retiré, 25 ml d'une suspension de poly(L-lysine hydrobromide) sont ensuite introduits dans le tube à une concentration finale de 20 mg/mL. La suspension obtenue est ensuite soniquée pendant 6 minutes à 4°C au doigt soniquant à 10 W, le tube est agité pendant 24 heures à 25°C sur une roue à une vitesse de 13 rotations par minute à une température comprise entre 4 et 8°C, la suspension est soniquée au doigt soniquant pendant 10 secondes à 10 W. Pour réaliser un premier lavage, le tube est alors placé contre le même aimant, le surnageant est retiré et remplacé par de l'eau MilliQ stérile. Les suspensions sont lavées de cette manière entre 1 et 4 fois. Enfin, la suspension obtenue est soniquée pendant 2 minutes à 24 W dans de la glace à une température inférieure à 4°C pour éviter un échauffement et le pH est ajusté à 6.8-7.2 avec du KOH filtrée. On obtient ainsi une suspension contenant des parties centrales des magnétosomes revêtues de poly-L-lysine.

**Caractérisation:** L'absorption de la suspension contenant 1 mg des parties centrales des magnétosomes revêtues de poly-L-lysine, mesurée à 480 nm, diminue d'environ 50% au bout de 20 minutes, ce qui indique la plus grande stabilité de cette suspension par rapport à celle contenant les parties centrales des magnétosomes non revêtues. Des images MET ont mis en évidence la présence d'un revêtement de poly-L-lysine autour des parties centrales de ces magnétosomes avec une épaisseur comprise entre 4 nm et 16 nm, une épaisseur moyenne de 8 nm ainsi qu'un un arrangement en chaînes d'une partie de ces nanoparticules synthétiques. Un test LAL, réalisé sur ces suspensions, révèle une faible concentration en endotoxines de 78 UE par millilitre par mg de fer (taux de recouvrement de 119%). Les mesures de diffusion de lumière, réalisées à l'aide du zétasizer, révèlent la présence de 92% d'agrégats de taille sphérique hydrodynamique 2489 nm et 8% d'objet sphérique ayant un diamètre hydrodynamique de 137 nm pouvant correspondre au diamètre hydrodynamique des parties centrales des magnétosomes revêtues de poly-L-lysine. Une analyse au CHNS révèle que l'échantillon contenant 10 mg des parties centrales des magnétosomes revêtues de poly-L-lysine contient des pourcentages en azote de 0,4%, en carbone de 3,6%, en hydrogène de 0,6%, en soufre de 0,03%. L'échantillon contenant 10 mg de poly-L-lysine lyophilisée contient un pourcentage en azote de 13%, en carbone de 33%, en hydrogène de 3,2%, en soufre de 0,03%. Ces résultats indiquent une plus faible concentration en carbone dans les suspensions contenant les parties centrales des magnétosomes, revêtues ou non de de poly-L-lysine, que dans celles contenant les bactéries entières ou les chaînes de magnétosomes pyrogènes extraites de MSR-1. Cela suggère la présence d'une moins grande quantité de matière organique dans les suspensions contenant les parties centrales des magnétosomes, revêtues ou non, que dans celles contenant les bactéries entières ou les chaînes de magnétosomes pyrogènes extraites de MSR-1. La présence du revêtement de poly-L-lysine autour des parties centrales des magnétosomes est suggérée par le pourcentage de carbone plus faible dans l'échantillon contenant les parties centrales des magnétosomes non revêtues (%C = 3.3%) que dans celui contenant les parties centrales des magnétosomes revêtues de poly-L-lysine (%C = 3.6%). Lorsque 1,5 mg d'une suspension contenant les parties centrales des magnétosomes revêtues de poly-L-lysine sont mélangés à 100 µl d'agar à 1% et que le mélange obtenu est soumis à l'application d'un champ magnétique alternatif d'intensité moyenne 32 mT et de fréquence 198 kHz, le mélange atteint une température de chauffage de 43°C au bout de 175 secondes. Des gels contenant 1,5 mg de nanoparticules chimiques (les BNF-Starch de Micromod, référence 10-00-102, et les M-PEI de Micromod, référence 17-00-152), mélangées à 100 µl d'agar à 1% sont soumis au même champ magnétique alternatif. Ils atteignent une température de 43°C au bout de 600 secondes, ce qui montre de meilleures propriétés de chauffage pour les suspensions contenant les parties centrales des magnétosomes revêtues de poly-L-lysine que pour celles contenant des nanoparticules chimiques BNF-Starch et M-PEI. Lorsque les parties centrales des magnétosomes revêtues de poly-L-lysine sont mélangées à des cellules GL-261 et que le mélange est soumis à la condition 3 de traitement, la température du mélange augmente de 11,5 °C, de 37 °C avant application du champ à 48,5 °C après 30 minutes d'application du champ. La pente à l'origine de la variation de température est estimée à 0,024 °C/sec. Lorsque ce même mélange est soumis à un traitement suivant la condition 2, le pourcentage de cellules vivantes est de 53 % alors qu'il est de seulement de 23% sans application du champ (condition 1 de traitement). Cela indique la cytotoxicité induite par les suspensions contenant les parties centrales des magnétosomes revêtues de poly-L-lysine sur les cellules GL261 en présence du champ magnétique.

**Cytotoxicité:** Un test de cytotoxicité au MTS (3-(4,5-dimethylthiazol-2-yl)-5-(3-carboxymethoxyphenyl)-2-(4-sulfophenyl)-2Htetrazolium) répondant à la norme ISO 10993-5, est effectué par un organisme agréé est effectué en conditions stériles sur des fibroblastes de souris L929 en utilisant des suspensions contenant les parties centrales des magnétosomes revêtues de poly-L-lysine à des concentrations de 0,01 mg/mL, 0,1 mg/mL, 0,5 mg/mL, et 1 mg/ml en fer contenues dans du milieu EMEM10. Ces suspensions sont d'abord incubées avec des cellules L929 à 37°C en présence de 5% de CO₂ pendant 24 à 26 heures. Après incubation, 20 µL d'une solution de coloration contenant du MTS et l'agent phenazine methosulfate (PMS) sont ajoutés aux cellules. Les cellules sont ensuite à nouveau incubées pendant 120 à 135 minutes à 37°C en présence de 5% de CO₂. La coloration, résultant en une absorption à 492 nm, permet de mettre en évidence la viabilité des cellules. Une observation des cellules au microscope permet de confirmer ou d'infirmer la viabilité des cellules. Les résultats obtenus montrent un pourcentage de cellules viables de 100%, 92% et 89% pour les suspensions contenant les parties centrales des magnétosomes revêtues de poly-L-lysine aux concentrations de 0,5 mg/mL, 0,1 mg/mL et 0,01 mg/mL en fer, cela indique l'absence de cytotoxicité de ces suspensions à ces concentrations. Pour une concentration de 1 mg/ml, il n'a pas été possible de conclure.

**Pyrogénicité:** Afin de confirmer l'absence de pyrogènicité de la suspension contenant les parties centrales des magnétosomes revêtues de poly-L-lysine, suggérée par les résultats du test LAL, un test pyrogène sur un lapin, répondant à la norme ISO10993-11, est effectué par un organisme agréé. Pour cela, la suspension contenant les parties centrales des magnétosomes revêtues de poly-L-lysine à une concentration en fer de 5 mg/ml a été placée au bain ultrason pendant 2 minutes, 1 ml de cette suspension a été dilué dans 119 ml de NaCl 0,9%. La température de la suspension a été maintenue à 37°C pendant 30 minutes, la suspension a été homogénéisée et administrée à trois lapins à une dose de 10 ml/kg par voie intraveineuse. La température corporelle des trois lapins a été mesurée toutes les 30 minutes pendant 3 heures. Elle augmente de 0,02°C (premier lapin), 0°C (deuxième lapin) et 0,22°C (troisième lapin). Aucun des trois lapins ne montre une augmentation de température supérieure à 0,5°C et la somme des élévations de température chez les trois lapins, qui est de 0,24°C, est inférieure à 2,65°C. Le produit testé n'est donc pas pyrogène suivant les critères des pharmacopées européennes et américaines.

**Toxicité aigüe:** Des tests de toxicité systémique aigue sont réalisés chez des souris femelles C57BL/6 âgées de 6 semaines en administrant 100 µl de suspensions contenant les parties centrales des magnétosomes revêtues de poly-L-lysine à une concentration de 0 mg, 0,5 mg, 1 mg, 2 mg, 4 mg et 8 mg de fer dans la queue des souris. Le poids corporel de chaque souris, mesuré quotidiennement pendant 12 jours après l'injection, reste stable, ce qui indique que la dose maximale tolérée par les souris est supérieure à 8 mg soit à environ 400 mg/Kg.

### Efficacité anti-tumorale sur des tumeurs GL261 implantées en sous-cutanée chez la souris:

A l'aide d'une seringue 25G de 1 mL, un volume de 50 µl contenant 2.10⁶ cellules de glioblastome murin GL261 est administré en sous-cutané sur le flanc gauche, entre la patte et le dos, de souris femelles black 6 C57BL/6 J. Les tumeurs croissent pendant 10 à 15 jours jusqu'à atteindre une taille comprise entre environ 40 et 150 mm³. Lorsque les tumeurs ont atteint cette taille, les souris sont anesthésiées à l'aide de gaz iso-flurane et maintenues à 37°C à l'aide de plaques chauffantes. A l'aide d'une seringue Hamilton de 250 µl, 50 µl de deux différentes suspensions apyrogènes sont administrés au centre des tumeurs contenant : (1), du glucose 5%, (2), les parties centrales des magnétosomes revêtues de poly-L-lysine à une concentration de 50 mg/mL en fer, mélangés à du glucose 5%. La suspension 2 est administrée à une quantité, mesurée en µg de fer, égale à 20.t, où t est la taille des tumeurs traitées en mm³. Ensuite, les souris sont (ou non) exposées pendant 30 minutes à un champ magnétique alternatif de fréquence 198 kHz et d'intensité moyenne variée entre 9 mT et 28 mT pour maintenir la température intratumorale à une valeur comprise entre 43 °C et 46 °C pendant les trois premières séances d'application du champ. Durant les séances suivantes d'application du champ, l'intensité moyenne du champ magnétique alternatif est fixée à 28 mT. La température intratumorale est mesurée à l'aide d'un thermocouple. Les séances d'application du champ magnétique sont répétées 3 fois par semaines, 15 fois en tout. Les souris dont les tumeurs continuent à augmenter suite à deux séances de chauffage subissent une seconde administration de la suspension (2). Les tailles de tumeurs sont mesurées à l'aide d'un pied à coulisse et les volumes tumoraux sont estimés en utilisant la formule Vₜᵤₘₒᵣₐₗ = 0,5.(L.l²), où L et 1 représentent respectivement la longueur et la largeur des tumeurs. Les souris sont euthanasiées quand le volume tumoral dépasse 1000 mm³ et/ou lorsque le poids des souris a diminué de plus de 20% d'une mesure à l'autre. Suivant ce protocole, des courbes de survie et de suivi du volume tumoral sont réalisées pendant 71 jours après l'administration des suspensions de nanoparticules.

On observe que 5 souris sur 10 traitées à l'aide des parties centrales des magnétosomes revêtues de poly-L-lysine et plusieurs applications du champ magnétique sont totalement guéries 15 jours après le début des séances de chauffage. Chez ces souris, il n'y a plus aucune trace visible de la tumeur ou de ses séquelles (croute ou cicatrice). Les 5 souris restantes traitées à l'aide des parties centrales des magnétosomes revêtues et application du champ magnétique, qui ne sont pas totalement guéries, sont euthanasiées environ 30 jours après le début du traitement. Les souris traitées avec les parties centrales des magnétosomes revêtues de poly-L-lysine sans application du champ magnétique sont toutes euthanasiées environ 12 jours après le début du traitement. Les taux de survie, 50 jours après le début du traitement, sont de 60% pour les souris traitées avec la suspension contenant les parties centrales des magnétosomes revêtues et application du champ magnétique alternatif et de 0% pour les souris traitées avec la suspension contenant les parties centrales des magnétosomes revêtues sans application du champ. Sans application du champ magnétique, toutes les souris sont mortes moins de 20 jours après le début du traitement.

### Efficacité anti-tumorale sur des tumeurs U87 implantées dans le cerveau de souris:

Les protocoles d'administration des cellules tumorales et de suivi des souris sont identiques à ceux décrits dans l'exemple 3. 6 jours après l'administration des cellules tumorales U87-Luc dans le cerveau de souris, lorsque les tumeurs atteignent une taille comprise entre 0.8 et 2 mm³, 2 µl de deux différentes suspensions sont administrés aux coordonnées (0.2.2) dans des cerveaux de souris anesthésiées. Pour les groupes 1 et 2, contenant 9 souris chacun, les souris reçoivent une suspension contenant les parties centrales des magnétosomes revêtues de poly-L-lysine à une concentration en fer de 250 mg/mL mélangées à 5% de glucose. Pour les groupes 3 et 4, contenant 9 souris chacun, les souris reçoivent une suspension contenant une solution injectable à 5% de glucose. Les souris des groupes 1 et 3 ne sont pas exposées à un champ magnétique. Celles des groupes 2 et 4 sont exposées pendant 30 minutes à un champ magnétique alternatif d'intensité moyenne 25 mT et de fréquence de 198 KHz trois fois par semaine pendant 6 semaines. Pour le groupe 2, 4 souris dont les tumeurs repoussent, reçoivent un traitement supplémentaire 8 semaines après implantation des cellules U87-Luc consistant en une deuxième administration de 2 µl de la suspension contenant les parties centrales des magnétosomes revêtues de poly-L-lysine à une concentration en fer de 147 mg/mL mélangées à 5% de glucose et en l'application du champ magnétique alternatif d'intensité moyenne 25 mT et de fréquence de 198 KHz trois fois par semaine pendant 3 semaines. Les variations de température sont mesurées lors des différents traitements à l'aide d'une caméra infra-rouge. Chez les souris traitées par administration des suspensions contenant les parties centrales des magnétosomes revêtues de poly-L-lysine et par de multiples applications du champ magnétique alternatif, une augmentation de température est observée pendant les seize premières séances d'application du champ pour les souris du groupe 2 qui est en moyenne de : 8°C (3 premières séances), 5°C (12 séances suivantes) et 1°C (16^{ème} séance). Aucune augmentation de température n'est observée lors des séances suivantes d'application du champ pour les souris du groupe 2. Pour les quatre souris du groupe 2 ayant reçu un traitement supplémentaire suite aux 16 séances de chauffage en raison de la non disparition totale de la tumeur, une augmentation de température de 5.5°C est observée pendant les 9 séances supplémentaires d'application du champ. Pour les groupes 1, 3 et 4, aucune augmentation de température n'est observée.

Chez les souris des groupes 3 et 4, les volumes tumoraux augmentent rapidement pour atteindre un volume moyen de 200 mm³ en moins de 45 jours suivant l'administration des cellules tumorales. Les temps de survie moyen des souris des groupes 3 et 4 ont été estimés à 40 jours en moyenne suivant l'administration des cellules tumorales, suggérant que la seule application du champ magnétique alternatif n'a pas d'effet anti-tumoral. Pour les souris du groupe 1, le volume tumoral augmente en moyenne de 5 mm³ par semaine, de manière beaucoup moins prononcée que pour les groupes 3 et 4. En outre, 13 semaines après l'implantation des cellules U87-Luc, 50% des souris du groupe 1 sont en vie, possédant un volume tumoral moyen de 56 mm³. Le traitement des souris du groupe 1 par la seule administration d'une suspension contenant les parties centrales des magnétosomes revêtues de poly-L-lysine permet un ralentissement de la croissance tumorale et une amélioration de la survie par rapport aux souris des groupes 3 et 4. Pour les souris du groupe 2, le volume tumoral moyen décroit durant les 85 jours suivant l'administration d'une suspension contenant les parties centrales des magnétosomes revêtues de poly-L-lysine jusqu'à la disparition totale de la tumeur qui est observée 42 jours suivant l'administration de ces suspensions (J42) pour 66% des souris. Pour les 4 souris du groupe 2 ayant reçu un second traitement en raison de la non disparition des tumeurs, la tumeur disparait totalement 20 jours après le début du second traitement. Globalement, 91 jours suivant l'administration des cellules U87-Luc, toutes les souris du groupe 2 sont encore en vie et ne présentent pas de signal de bioluminescence, ce qui suggère leur guérison.

Nous pouvons conclure que : (i) lorsque les suspensions contenant les parties centrales des magnétosomes revêtues de poly-L-lysine sont administrées dans des tumeurs U87-Luc, la croissance tumorale est ralentie, ce qui pourrait s'expliquer par la forte concentration en fer de ces suspensions ou par la présence de poly-L-lysine, (ii) lorsque ces mêmes suspensions sont administrées dans les tumeurs U87-Luc et soumises à de multiples application d'un champ magnétique alternatif de fréquence 198 kHz et d'intensité moyenne 25 mT, il est possible de faire disparaitre entièrement ces tumeurs chez la totalité des souris en moins de 4 mois.

### Exemple 7: Parties centrales des magnétosomes issues de MSR-1, revêtues de chitosan.

**Préparation:** Une suspension contenant 1 mg de la partie centrale des magnétosomes mélangée à de l'eau apyrogène stérile est soniquée pendant 5 minutes au doigt soniquant à une puissance de 5W avec des pulses de 0,1 seconde, séparés d'intervalles de 0,1 seconde. 0,25 mg de chitosan (chitosan hydrochloré CRS, référence sigma Y0000104) est mélangé à la suspension contenant 1 mg de la partie centrale des magnétosomes et le pH de la suspension est ajusté à 6,2 avec de la soude 1 M. Le mélange obtenu est soniqué au doigt soniquant à une puissance de 5 W, avec des pulses de 0,1 seconde, séparés d'intervalles de 0,1 seconde, pendant 60 minutes à 45 °C, puis pendant 15 minutes à 60 °C, puis pendant 15 minutes à 70 °C. La suspension obtenue est lavée une première fois en plaçant un aimant de Néodinium contre la paroi du tube en verre qui contient la suspension, en retirant le surnageant et en le remplaçant par de l'eau apyrogène stérile. Elle est lavée une deuxième fois de la même manière.

**Caractérisation:** Le taux d'endotoxines de ces magnétosomes revêtus de chitosan est de 25 UE/mg/mL. L'absorption, mesurée à 480 nm, d'une suspension contenant 1 mg des parties centrales des magnétosomes revêtues de chitosan diminue de 3% durant les 20 minutes de mesure, ce qui montre la stabilité de cette suspension. Les mesures MET effectuées sur les parties centrales des magnétosomes revêtues de chitosan indiquent un arrangement de ces nanoparticules synthétiques en chaînes, la présence d'agrégats et d'un revêtement entourant les parties centrales des magnétosomes d'épaisseur moyenne ∼6 nm. Lorsqu'une suspension contenant les parties centrales des magnétosomes revêtues de chitosan est mélangée à des cellules GL-261 et que le mélange est soumis à la condition 3 de traitement, la température du mélange augmente de 8,5 °C. La pente à l'origine de la variation de température est estimée à 0,009 °C/sec lors d'une première série de mesures. Lorsque ce même mélange est soumis à la condition 2 de traitement, le pourcentage de cellules vivantes est de 0-5% alors qu'il est de 20% en absence de champ (condition 1 de traitement). Cela montre l'efficacité de destruction des cellules tumorales des parties centrales des magnétosomes revêtues de chitosan dans la condition 2 de traitement. Le point isoélectrique de ces magnétosomes est estimé à pH 11. Le potentiel zéta varie entre 46 mV à pH 2 à -55 mV à pH 12. Les tailles hydrodynamiques de ces magnétosomes sont estimées à 273 nm pout 7% d'entre eux et 1908 nm pour 93% d'entre eux. L'analyse au CHNS indique que leur pourcentage de carbone est estimé à 3,2%, proche du pourcentage de carbone de 3,3% mesuré pour les magnétosomes non revêtus.

### Exemple 8 : Parties centrales des magnétosomes issues de MSR-1, revêtues de carboxy-méthyldextran.

**Préparation:** Une suspension contenant 1 mg de la partie centrale des magnétosomes mélangée à de l'eau apyrogène stérile est soniquée pendant 5 minutes au doigt soniquant à une puissance de 5W avec des pulses de 0,1 seconde, séparés d'intervalles de 0,1 seconde. 4 mg de carboxy-methyl dextran (référence SIGMA 86524-10G-F) sont mélangés à 1 mg de la partie centrale des magnétosomes et le pH du mélange est ajusté à 3,5 avec de l'acide chlorhydrique 1M. Le mélange obtenu est soniqué pendant 60 minutes à 45°C au doigt soniquant à une puissance de 5W, avec des pulses de 0,1 seconde, séparés d'intervalles de 0,1 seconde. La suspension obtenue est lavée trois fois. Pour le premier lavage, un aimant est placé contre la paroi du tube, le surnageant est retiré et remplacé par de l'eau apyrogène stérile. La suspension obtenue est lavée une deuxième, puis une troisième fois de la même manière.

**Caractérisation:** Lors d'une première série de mesures, l'absorption, mesurée à 480 nm, d'une suspension contenant 1 mg des parties centrales des magnétosomes revêtues de carboxy-methyldextran ne diminue pas durant les 20 minutes de mesure, ce qui montre la stabilité de cette suspension. Les mesures MET ont permis d'estimer une épaisseur du revêtement entourant les parties centrales des magnétosomes comprise entre 2 et 20 nm. Les mesures de diffusion de lumière des suspensions contenant les parties centrales des magnétosomes revêtues de carboxy-méthyldextran révèlent la présence de 79% d'objets sphériques de diamètre hydrodynamique 1359 nm (population 1), 6% d'objets sphériques de diamètre hydrodynamique 5124 nm (population 2) et 15% d'objets sphériques de diamètre hydrodynamique 331 nm (population 3). La taille de la population 3 pourrait correspondre à celle des parties centrales des magnétosomes revêtues de carboxy-méthyldextran. Le point isoélectrique est estimé à pH=3,4 et le potentiel zéta diminue de 20 mV à pH=2 à -31 mV à pH=12. Lorsqu'une suspension contenant les parties centrales des magnétosomes revêtues de carboxy-methyldextran est mélangée à des cellules GL-261 et que le mélange est soumis à la condition 3 de traitement, la température du mélange augmente de 29 °C. La pente à l'origine de la variation de température est estimée à 0,023 °C/sec. Lorsque ce même mélange est soumis à la condition 2 de traitement, le pourcentage de cellules vivantes est de 11% alors qu'il est de 63% en absence de champ (condition 1 de traitement). Cela montre l'efficacité de destruction des cellules tumorales des parties centrales des magnétosomes revêtues d'acide citrique dans la condition 2 de traitement. Le pourcentage de carbone mesuré au CHNS dans ces magnétosomes est de 3,7%.

### Exemple 9: Parties centrales des magnétosomes issues de MSR-1, revêtues d'acide citrique.

**Préparation:** Une suspension contenant 50 mg des parties centrales des magnétosomes mélangés à 4,5 mL d'eau apyrogène stérile est soniquée pendant 5 minutes au doigt soniquant à une puissance de 5W, avec des pulses de 0,1 seconde, séparés d'intervalles entre pulses de 0,1 seconde. 20 mg de cette suspension, contenus dans un volume de 1,8 mL, sont mélangés à 35 mg d'acide citrique monohydrate (référence Sigma 33114-500G) contenu dans 8 mL d'eau apyrogène stérile. Lors de cette préparation, la masse de fer est 1,75 fois plus élevée que la masse d'acide citrique. Le pH de la suspension est ajusté à 6 avec de la soude 1 M, le mélange obtenu est soniqué à 90°C pendant une heure. La suspension obtenue est lavée une première fois en plaçant un aimant de Néodinium contre la paroi du tube en verre, en retirant le surnageant et en le remplaçant par de l'eau apyrogène stérile. Elle est lavée une deuxième fois de la même manière.

**Caractérisation:** L'absorption, mesurée à 480 nm, d'une suspension contenant 1 mg des parties centrales des magnétosomes revêtues d'acide citrique, diminue d'environ 15% en 20 minutes, ce qui montre la stabilité de cette suspension. Les images MET de ces nanoparticules synthétiques révèlent la présence de nombreuses chaînes, une bonne dispersion de ces nanoparticules synthétiques, peu d'agrégation et la présence d'un revêtement autour de la partie centrale de ces magnétosomes d'épaisseur comprise entre 1 et 15 nm. Un test LAL, réalisé sur ces suspensions, révèle une faible concentration en endotoxines de 19 UE par millilitre par mg de fer (taux de recouvrement de 188%). Une analyse au CHNS, réalisée sur ces suspensions lyophilisées, révèle un pourcentage d'azote de 0,8%, de carbone de 3,7%, d'hydrogène de 0,3% et l'absence de soufre. L'échantillon contenant uniquement l'acide citrique lyophilisé contient un pourcentage d'azote de 0%, de carbone de 36%, d'hydrogène de 4,7% et pas de soufre. Ces résultats indiquent un plus faible pourcentage en carbone dans les suspensions contenant les parties centrales des magnétosomes, revêtues ou non d'acide citrique, que dans celles contenant les bactéries entières ou les chaînes de magnétosomes pyrogènes extraites de MSR-1. Cela peut également suggérer la présence d'une moins grande quantité de matière organique dans les suspensions contenant les parties centrales des magnétosomes, revêtues ou non d'acide citrique, que dans celles contenant les bactéries entières ou les chaînes de magnétosomes pyrogènes extraites de MSR-1. La présence du revêtement autour des parties centrales des magnétosomes revêtues d'acide citrique est suggérée par le plus faible pourcentage de carbone dans la suspension lyophilisée contenant la partie centrale des magnétosomes (%C = 3.3%) que dans la suspension lyophilisée contenant les parties centrales des magnétosomes revêtues d'acide citrique (%C = 3.7%). Lorsqu'une suspension contenant les parties centrales des magnétosomes revêtues d'acide citrique est mélangée à des cellules GL-261 et que le mélange est soumis à la condition 3 de traitement, la température du mélange augmente de 25 °C, de 35°C avant application du champ à 60 °C après application du champ. La pente à l'origine de la variation de température est estimée à 0,038 °C/sec. Lorsque ce même mélange est soumis à la condition 2 de traitement, le pourcentage de cellules vivantes est de 26% alors qu'il est de 57% en absence de champ (condition 1 de traitement). Cela montre l'efficacité de destruction des cellules tumorales des parties centrales des magnétosomes revêtues d'acide citrique dans la condition 2 de traitement. Les mesures de diffusion de lumière de ces suspensions révèlent la présence d'objets non-sphériques, pouvant correspondre à des chaînes, de taille hydrodynamique 788 nm. Le point isoélectrique est estimé à pH=3,7 et le potentiel zéta diminue de 25 mV à pH=2 à -38 mV à pH=12.

### Exemple 10: Parties centrales des magnétosomes issues de MSR-1, revêtues d'acide oléique, préparées suivant le premier protocole.

**Préparation:** On ajoute à 5 mL d'une suspension contenant 5 mg de la partie centrale des magnétosomes de concentration 1 mg/mL en fer, 10 µL d'une solution d'ammoniaque NH₄OH (25% en poids moléculaire) afin d'obtenir un pH compris entre 10 et 11. On sonique la suspension obtenue dans un bain-marie à 80°C pendant 15 minutes avec un doigt soniquant à une puissance de 6-7 W avec des pulses de 0,2 secondes, séparés d'intervalle de 0,5 secondes. Ensuite, on ajoute soit 267 µl d'une solution d'acide oléique à 211 mM (condition 1) soit 50 µL d'une solution d'acide oléique à 32 mM (0.4 mg) (condition 2). Puis on sonique le mélange au doigt soniquant pendant 1 heure à une puissance de 6-7 W, avec des pulses de 0,2 secondes, séparés d'intervalle entre pulses de 0,5 secondes. La suspension obtenue est ensuite lavée à l'aide d'un aimant au Néodinium qui est placé contre le tube en verre contenant la suspension, le surnageant est retiré et remplacé par de l'eau apyrogène stérile. La suspension est lavée une deuxième fois, puis une troisième et une quatrième fois de la même manière. Une fois le dernier lavage réalisé, on prélève des aliquotes afin de réaliser les différents tests de caractérisation. On conserve la suspension à 4°C jusqu'à son utilisation.

**Caractérisation:** L'absorption, mesurée à 480 nm, d'une suspension contenant les parties centrales des magnétosomes revêtues d'acide oléique, ne diminue pas pendant 20 minutes, indiquant sa stabilité. Des images MET des parties centrales des magnétosomes revêtues d'acide oléique, préparées suivant la condition 2, ont mis en évidence la présence d'agrégats de nanoparticules et d'un revêtement autour de la partie centrale des magnétosomes d'épaisseur de 0,5 à 5 nm. Les mesures de diffusion de lumière, réalisées sur ces magnétosomes revêtus, synthétisés suivant la condition 2, révèlent la présence d'objets sphériques stables, ne s'agrégeant pas, de diamètre hydrodynamique 123 nm pouvant correspondre au diamètre hydrodynamique de ces magnétosomes revêtus. Le point isoélectrique est estimé à pH=3,5 et le potentiel zéta monte la présence de deux populations dont le potential zéta diminue pour l'une de 30 mV à pH=2 à -60 mV à pH=12 et pour l'autre de -10 mV à pH=6 à -35 mV à pH=12. Lorsqu'une suspension contenant les parties centrales des magnétosomes revêtues d'acide citrique est mélangée à des cellules GL-261 et que le mélange est soumis à la condition 3 de traitement, la température du mélange augmente de 28 °C. La pente à l'origine de la variation de température est estimée à 0,051 °C/sec.

### Exemple 11: Parties centrales des magnétosomes issues de MSR-1, revêtues d'acide oléique, préparées suivant le second protocole.

**Préparation:** La partie centrale peut être revêtue d'acide oléique de la manière suivante. On ajoute à 1 mL d'une suspension contenant 10 mg de la partie centrale des magnétosomes de concentration 1 mg/mL en fer, 100 mg d'une solution d'acide oléique à 10 mg/mL à pH 11. On sonique la suspension obtenue pendant 5 minutes avec un doigt sonicant à une puissance de 20 W, en continue et à température ambiante. Ensuite, la suspension est congelée au -80°C pendant 30 minutes puis chauffée à 80°C pendant 5 minutes. Puis on sonique le mélange au doigt sonicant pendant 1,5 heure à une puissance de 10 W, avec des pulses de 3 secondes, séparés d'intervalle entre pulses de 3 secondes. La suspension obtenue est ensuite lavée à l'aide d'un aimant au Néodinium qui est placé contre le tube en verre contenant la suspension, le surnageant est retiré et remplacé par de l'eau apyrogène stérile. La suspension est lavée une deuxième fois et une troisième fois de la même manière. Une fois le dernier lavage réalisé, on prélève des aliquotes afin de réaliser les différents tests de caractérisation. On conserve la suspension à 4°C jusqu'à son utilisation.

**Caractérisation** : Les propriétés des parties centrales revêtues d'acide oléique peuvent être les suivantes. L'absorption, mesurée à 480 nm, d'une suspension des parties centrales des magnétosomes revêtues d'acide oléique contenant 1 mg en fer, ne diminue pas pendant 20 minutes, indiquant sa stabilité. Lorsqu'une suspension contenant les parties centrales des magnétosomes revêtues d'acide oléique est mélangée à des cellules GL-261 et que le mélange est soumis à la condition 3 de traitement, la température du mélange augmente de 5 °C. La pente à l'origine de la variation de température est estimée à 0,012 °C/sec. Lorsque ce mélange est soumis à la condition 2 de traitement, le pourcentage de cellules vivantes est de 14% alors qu'il est de 53% en absence de champ (condition 1 de traitement). Le pourcentage de carbone dans ces magnétosomes est de 3,4%.

### Exemple 12: Parties centrales des magnétosomes issues de MSR-1, revêtues d'acide folique.

**Préparation:** Une suspension contenant 50 mg en fer des parties centrales des magnétosomes mélangés à 4,5 mL d'eau apyrogène stérile est soniquée pendant 5 minutes au doigt soniquant à une puissance de 30 W, avec des pulses de 30 secondes, séparés d'intervalles de 10 secondes. Un volume de 1,8 mL, contenant 20 mg en fer des parties centrales des magnétosomes, est introduit dans un tube en verre stérile de 10 mL placé contre un aimant, le surnageant est retiré et remplacé par 8 mL d'une solution à 2 mg/mL en acide folique (Fisher BioReagents ; référence : 59-30-3) mélangé avec de l'eau apyrogène stérile, préalablement ajusté à pH 9.5 avec une solution à 1 M d'hydroxyde de sodium et stérilisé par filtration (filtre 0.45µm). Lors de cette préparation, la masse de fer est 1,25 fois plus élevée que la masse d'acide folique. Le mélange obtenu est soniqué pendant 1.5 heure au doigt soniquant à une puissance de 30 W avec des pulses de 30 secondes, séparés d'intervalles entre pulses de 10 secondes. La suspension obtenue de volume 8 mL est contenue dans un tube en verre de 10 mL. Elle est lavée une première fois en plaçant un aimant de Néodinium contre la paroi du tube en verre, en retirant le surnageant puis en le remplaçant par de l'eau apyrogène Hyclone et en soniquant pendant 1 minute à 30W. Elle est lavée quatre fois supplémentaires de la même manière.

**Caractérisation:** L'absorption, mesurée à 480 nm, d'une suspension contenant 1 mg des parties centrales des magnétosomes revêtues d'acide folique ne diminue pas pendant 20 minutes, ce qui montre la stabilité de cette suspension. Les mesures de diffusion de cette suspension révèlent la présence de 90% d'agrégats sphériques de diamètre hydrodynamique 2876 nm et de 10% d'objets sphériques de diamètre hydrodynamique 235 nm qui pourraient correspondre aux magnétosomes revêtus d'acide folique. Le point isoélectrique de cette suspension a été estimé à pH 7,9 et le potentiel zéta diminue de 45 mV à pH 2 à -43 mV à pH 12. L'épaisseur du revêtement entourant les parties centrales de ces magnétosomes a été mesurée à une valeur comprise entre 1 et 4 nm. Lorsqu'une suspension contenant les parties centrales des magnétosomes revêtues d'acide citrique est mélangée à des cellules GL-261 et que le mélange est soumis à la condition 3 de traitement, la température du mélange augmente de 20 °C. La pente à l'origine de la variation de température est estimée à 0,042 °C/sec. Lorsque ce même mélange est soumis à la condition 2 de traitement, le pourcentage de cellules vivantes est de 9% alors qu'il est de 93% en absence de champ (condition 1 de traitement). Cela montre l'efficacité de destruction des cellules tumorales des parties centrales des magnétosomes revêtues d'acide citrique dans la condition 2 de traitement. Le pourcentage de carbone dans ces magnétosomes est estimé à 3,9%.

### Exemple 13: Parties centrales des magnétosomes issues de MSR-1, revêtues de DOPC.

**Préparation:** Dans un tube en verre de 10 mL, 40 mg de DOPC (1,2-Dioleoyl-sn-glycero-3-phosphocholine, référence Sigma : P6354) ont été solubilisé dans 100 µL de chloroforme puis le solvant a ensuite été évaporé pendant 10 minutes à l'aide de gaz inerte (azote) pour former un film lipidique homogène dans le tube. Une suspension de parties centrales de magnétosomes contenant 20 mg de fer, mélangés à 8 mL d'eau apyrogène stérile, est introduite dans le tube contenant le film lipidique puis elle est soniquée pendant 1.5 heures au doigt soniquant à une puissance de 30 W avec des pulses de 10 secondes, séparés par des intervalles de 0.5 secondes. Lors de cette préparation, la masse de DOPC est 2 fois plus élevée que la masse de fer. La suspension obtenue est lavée une première fois en plaçant un aimant de Néodinium contre la paroi du tube en verre, en retirant le surnageant puis en le remplaçant par de l'eau apyrogène stérile et en soniquant pendant 1 minute à 30W. Elle est lavée cinq fois supplémentaires de la même manière.

**Caractérisation:** Lors d'une première série de mesures, l'absorption, mesurée à 480 nm, d'une suspension contenant les parties centrales des magnétosomes revêtues de DOPC ne diminue pas pendant les 20 minutes de mesure, ce qui montre la stabilité de cette suspension. Les images MET indiquent la présence d'un revêtement entourant les parties centrales de ces magnétosomes d'épaisseur 0,6 à 3 nm. Les mesures de diffusion de lumière, réalisées sur ces suspensions, révèlent la présence de 87% d'agrégats sphériques de diamètre hydrodynamique 1871 nm et de 13% d'objets sphériques de diamètre hydrodynamique 278 nm qui pourraient correspondre aux tailles des parties centrales des magnétosomes revêtues de DOPC. Le point isoélectrique est mesuré à pH 3 et le potentiel zéta diminue de 10 mV à pH 2 à -35 mV à pH 12. Lorsqu'une suspension contenant les parties centrales des magnétosomes revêtues de DOPC est mélangée à des cellules GL-261 et que le mélange est soumis à la condition 3 de traitement, la température du mélange augmente de 33,5°C. La pente à l'origine de la variation de température est estimée à 0,05 °C/sec. Lorsque ce même mélange est soumis à la condition 2 de traitement, le pourcentage de cellules vivantes est de 0-5% alors qu'il est de 13% sans application du champ (condition 1 de traitement). Cela indique la cytotoxicité induite par les parties centrales des magnétosomes revêtues de DOPC sur les cellules GL261 en présence d'un champ magnétique alternatif. Le pourcentage de carbone dans ces magnétosomes est estimé à 7,5%.

### Exemple 14: Parties centrales des magnétosomes issues d'AMB-1, revêtues de DOPC.

**Préparation:** Dans un tube conique en verre de 35 mL, 280 mg de DOPC (1,2-Dioleoyl-sn-glycero-3-phosphocholine, référence Sigma : P6354) sont solubilisés dans 500 µL de chloroforme puis le solvant est évaporé pendant 15 minutes à l'aide d'azote pour former un film lipidique homogène dans le tube. Une suspension de parties centrales de magnétosomes, contenant 10 mg en fer mélangés à 15 mL d'eau apyrogène stérile, est introduite dans le tube contenant le film lipidique puis est soniquée pendant 30 minutes au doigt soniquant à une puissance de 20 W avec des pulses de 10 secondes, séparés par des intervalles entre pulses de 20 secondes. Lors de cette préparation, la masse de DOPC est 28 fois plus élevée que la masse de fer. La suspension obtenue est purifiée à l'aide d'une colonne d'exclusion de taille SEPHADEX G-25 (GE Healthcare, Buckimghamshire, UK). La suspension de couleur marron est collectée puis elle est concentrée en plaçant un aimant de Néodinium contre la paroi du tube en verre, en retirant une partie du surnageant qui est remplacé par de l'eau stérile apyrogène et en soniquant l'échantillon pendant 1 minute à 30W.

**Caractérisation** : Les images MET de ces suspensions permettent d'estimer une épaisseur du revêtement comprise entre 50 et 150 nm.

### Exemple 15: Parties centrales des magnétosomes issues d'AMB-1, revêtues d'alendronate.

**Préparation:** Un volume de 2 mL, contenant 10 mg de fer, a été introduit dans un tube en verre (apyrogène) de 25 mL puis ce dernier a été placé contre un aimant. Le surnageant a été éliminé puis remplacé par 10 mL d'une solution à 11,6 mg/mL en alendronate mélangé avec de l'eau MilliQ stérile, préalablement ajusté à pH 2.5 avec une solution à 0.1 M d'acide chlorhydrique et stérilisé par filtration (filtre 0.45µm). Lors de cette préparation, la masse d'alendronate est 11,6 fois plus élevée que la masse de fer. Le mélange obtenu est soniqué en continue pendant 15 minutes au doigt soniquant avec une puissance de 30 W. La suspension est ensuite chauffée sous micro-onde à 70W, 3 fois 1 minute avec un intervalle de 5 min où l'échantillon est refroidit avec un bain de glace. La suspension est lavée une première fois en plaçant un aimant de Néodinium contre la paroi du tube en verre, en retirant le surnageant puis en le remplaçant par de l'eau MilliQ stérile et en soniquant pendant 1 minute à 30W. Elle est lavée dix fois de la même manière en plaçant un aimant de Néodinium contre la paroi du tube en verre, en retirant le surnageant puis en le remplaçant par de l'eau MilliQ stérile et en soniquant pendant 1 minute à 30W.

**Caractérisation:** Les mesures du test LAL permettent d'estimer une quantité en endotoxines dans ces suspensions comprise entre 53 et 144 UE/mg/mL. L'absorption, mesurée à 480 nm, d'une suspension des parties centrales des magnétosomes revêtues d'alendronate contenant 1 mg de fer diminue de 1% pas pendant les 20 minutes de mesure, ce qui montre la stabilité de cette suspension. Des mesures MET indiquent qu'une matrice entoure ces magnétosomes. Ces magnétosomes possèdent un point isoélectrique de pH 3,5, des tailles hydrodynamiques de 527 nm pou 14% d'entre eux et de 2735 nm pour 86% d'entre eux, un potentiel zéta qui varie de 25 mV à pH 2 à -47 mV à pH 12. Lorsqu'une suspension contenant les parties centrales des magnétosomes revêtues d'alendronate est mélangée à des cellules GL-261 et que le mélange est soumis à la condition 3 de traitement, la température du mélange augmente de 18,3°C. La pente à l'origine de la variation de température est estimée à 0,28 °C/sec. Lorsque ce même mélange est soumis à la condition 2 de traitement, le pourcentage de cellules vivantes est de 2% alors qu'il est de 17% sans application du champ (condition 1 de traitement). Cela indique la cytotoxicité induite par les parties centrales des magnétosomes revêtues d'alendronate sur les cellules GL261 en présence d'un champ magnétique alternatif. Le pourcentage de carbone dans ces magnétosomes est estimé à 9%.

### Exemple 16: Parties centrales des magnétosomes issues de MSR-1, revêtues de néridronate.

**Préparation** : Une suspension contenant 50 mg en fer des parties centrales des magnétosomes, mélangée à 4,5 mL d'eau apyrogène stérile, est soniquée pendant 5 minutes au doigt soniquant à une puissance de 30 W, avec des pulses de 30 secondes, séparés par des intervalles entre pulses de 10 secondes. Un volume de 1,8 mL, contenant 20 mg en fer de la suspension obtenue, est introduit dans un tube en verre de 10 mL placé contre un aimant, le surnageant de la suspension est retiré et remplacé par 8 mL d'une solution à 20 mg/mL en néridronate mélangé avec de l'eau apyrogène stérile et dont le pH est ajusté à 2.5. Lors de cette préparation, la masse de néridronate est 8 fois plus élevée que la masse de fer. Le mélange obtenu est soniqué pendant 2 heures au doigt soniquant à une puissance de 30 W, avec des pulses de 30 secondes, séparés par des intervalles de 10 secondes. La suspension est lavée une première fois en plaçant un aimant de Néodinium contre la paroi du tube en verre, en retirant le surnageant puis en le remplaçant par de l'eau apyrogène stérile préalablement ajusté à pH 11. Elle est lavée cinq fois supplémentaires de la même manière.

**Caractérisation:** L'absorption, mesurée à 480 nm, de la suspension contenant les parties centrales des magnétosomes revêtues de néridronate ne diminue pas pendant 20 minutes, indiquant la stabilité de cette suspension. Les mesures MET indiquent la présence d'un revêtement autour des parties centrales des magnétosomes avec une épaisseur comprise entre 19 et 200 nm. Les mesures de diffusion de lumière, réalisées sur ces suspensions, indiquent la présence d'1% d'agrégats sphériques de diamètre hydrodynamique 5560 nm, de 59% d'agrégats sphériques de diamètre hydrodynamique 710 nm et de 40% d'objets sphériques de diamètre hydrodynamique 207 nm pouvant correspondre aux parties centrales des magnétosomes revêtues de néridronate. Lorsqu'une suspension contenant les parties centrales de magnétosomes revêtues de néridronate est mélangée à des cellules GL-261 et soumise à la condition 3 de traitement, la température du mélange augmente de 37,2 °C avant application du champ à 43,9 °C après 30 minutes d'application du champ. La pente à l'origine de la variation de température est estimée à 0,017 °C/sec. Lorsque ce même mélange est soumis à la condition 2 de traitement, le pourcentage de cellules vivantes est de 28% alors qu'il est de 44% sans application du champ (condition 1 de traitement). Cela indique la cytotoxicité induite par les parties centrales des magnétosomes revêtus de néridronate sur les cellules GL261 en présence de la condition 2 de traitement. Le point isoélectrique de ces nanoparticules synthétiques revetues de néridronate est estimé à pH 3,5 et le potentiel zéta diminue de 40 mV à pH 2 à -42 mV à pH 12. Le pourcentage de carbone de ces magnétosomes est estimé à 18,1 %.

### Exemple 17: Parties centrales des magnétosomes issues de MSR-1, revêtues de PEI.

**Préparation:** Une suspension de minéraux de magnétosomes contenant 50 mg de fer mélangés à 4,5 mL d'eau apyrogène stérile est soniquée pendant 5 minutes au doigt soniquant à une puissance de 30 W, avec des pulses de 30 secondes, séparés par des intervalles de 10 secondes. Un volume de 1,8 mL, contenant 20 mg de fer de cette suspension, est introduit dans un tube en verre de 10 mL placé contre un aimant. Le surnageant est éliminé et remplacé par 8 mL d'une solution à 25 mg/mL en PEI, mélangé à de l'eau apyrogène stérile de pH préalablement ajusté à 9.5. Lors de cette préparation, la masse de PEI est 10 fois plus élevée que la masse de fer. Le mélange obtenu est soniqué pendant 2 heures au doigt soniquant à une puissance de 30 W, avec des pulses de 30 secondes, séparés par des intervalles de 10 secondes. La suspension obtenue est lavée une première fois en plaçant un aimant de Néodinium contre la paroi du tube en verre, en retirant le surnageant, en le remplaçant par de l'eau apyrogène stérile et en soniquant pendant 1 minute à 30W. Elle est lavée cinq fois supplémentaires de la même manière.

**Caractérisation:** L'absorption de la suspension contenant les parties centrales des magnétosomes revêtues de PEI, mesurée à 480 nm, ne diminue pas pendant les 20 minutes de mesure, ce qui montre la stabilité de cette suspension. Les mesures MET indiquent une épaisseur de revêtement de 8 à 10 nm. Les mesures de diffusion de lumière de cette suspension indiquent la présence d'objets sphériques de diamètre hydrodynamique 175 nm qui pourraient correspondre aux parties centrales des magnétosomes revêtues de PEI. Le point isoélectrique est estimé à 11 et le potentiel zéta diminue de 42 mV à pH 2 à -16 mV à pH 12. Les mesures au CHNS révèlent un pourcentage d'azote de 1,1% et un pourcentage de carbone de 4,5%, tous deux supérieurs aux pourcentages d'azote de 0,2% et de carbone de 3,3% présents dans les parties centrales des magnétosomes non revêtues, suggérant la présence du revêtement. Lors d'une deuxième série de mesures, lorsqu'une suspension contenant les parties centrales de magnétosomes revêtues de PEI est mélangée à des cellules GL-261 et soumise à la condition 3 de traitement, la température du mélange augmente de 37 °C avant application du champ à 43°C après 30 minutes d'application du champ. La pente à l'origine de la variation de température est estimée à 0,014 °C/sec. Lorsque ce même mélange est soumis à la condition 2 de traitement, le pourcentage de cellules vivantes est de 15% alors qu'il est de 40% sans application du champ (condition 1 de traitement). Cela indique la cytotoxicité induite par les parties centrales des magnétosomes revêtus de PEI sur les cellules GL261 en présence de la condition 2 de traitement.

### Exemple 18: Parties centrales des magnétosomes issues d'AMB-1, revêtues de PEI

**Préparation:** Une suspension contenant 10 mg en fer des parties centrales des magnétosomes, mélangée à 10 mL d'eau apyrogène stérile est soniquée pendant 5 minutes au doigt soniquant à 30 W avec des pulses de 30 secondes, séparés par des intervalles de 10 secondes. Cette suspension est placée contre un aimant, le surnageant est retiré puis remplacé par 10 mL d'une solution à 20 mg/mL en PEI, mélangé à de l'eau apyrogène stérile à pH 9.5. Lors de cette préparation, la masse de PEI est 2 fois plus élevée que la masse de fer. Le mélange obtenu est soniqué pendant 30 minutes au doigt soniquant à une puissance de 20 W avec des pulses de 10 secondes, séparés par des intervalles entre pulses de 20 secondes. La suspension est refroidie toutes les deux minutes à l'aide d'un bain de glace. Elle est lavée une première fois en plaçant un aimant de Néodinium contre la paroi du tube en verre, en retirant le surnageant puis en le remplaçant par de l'eau apyrogène Hyclone et en soniquant pendant 1 minute à 30W. Elle est lavée dix fois supplémentaires de la même manière.

**Caractérisation:** Le test LAL révèle une concentration en endotoxines inférieure à 50 UE/mg/mL dans ces suspensions. Les mesures MET permettent d'estimer une épaisseur du revêtement entourant les parties centrales de ces magnétosomes comprise entre 4 et 18 nm. L'absorption de la suspension des parties centrales des magnétosomes revêtues de PEI contenant 1 mg de fer, mesurée à 480 nm, diminue de 64% pendant les 20 minutes de mesure. Les mesures de diffusion de lumière de cette suspension indiquent la présence d'objets de tailles hydrodynamiques 125 nm pour 6% d'entre eux, 5445 nm pou 1% d'entre eux et 1067 nm pour 93% d'entre eux. Le point isoélectrique est estimé à 11,3 et le potentiel zéta diminue de 50 mV à pH 2 à -10 mV à pH 12. Les mesures au CHNS révèlent un pourcentage de carbone de 6,6%. Lorsqu'une suspension contenant les parties centrales de magnétosomes revêtues de PEI est mélangée à des cellules GL-261 et soumise à la condition 3 de traitement, la température du mélange augmente de 12 °C après 30 minutes d'application du champ. La pente à l'origine de la variation de température est estimée à 0,04 °C/sec. Lorsque ce même mélange est soumis à la condition 2 de traitement, le pourcentage de cellules vivantes est de 12% alors qu'il est de 26% sans application du champ (condition 1 de traitement). Cela indique la cytotoxicité induite par les parties centrales des magnétosomes revêtus de PEI sur les cellules GL261 en présence de la condition 2 de traitement.

### Exemple 19: Parties centrales des magnétosomes issues de MSR-1, revêtues d'Al(OH)₃

**Préparation:** La suspension contenant 7 mg par millilitre de la partie centrale des magnétosomes est d'abord soniquée pendant 5 minutes à une puissance de 5 W avec des pulses de 0,1 seconde et des intervalles entre pulses de 0,1 seconde. Dans un tube en verre de 8 mL, une suspension contenant 2 mg de la partie centrale des magnétosomes est introduite, la suspension est placée contre un aimant au néodyme, le surnageant est retiré et remplacé par 600 µl d'hydroxyde d'aluminium alhydrogen à 10 mg/mL. Le mélange est soniqué pendant 90 minutes en continu à 20 W. On effectue un premier lavage en plaçant la suspension contre un aimant, en retirant le surnageant et en le remplaçant par de l'eau hyclone. On effectue trois lavages supplémentaires de la même manière.

**Caractérisation:** L'absorption de la suspension contenant les parties centrales des magnétosomes revêtues d'Al(OH)₃, mesurée à 480 nm, ne diminue pas pendant les 20 minutes de mesure, ce qui montre la stabilité de cette suspension. Les mesures de microscopie électronique révèlent la présence d'un gel entourant les parties centrales de ces magnétosomes. Les mesures de diffusion de lumière de cette suspension indiquent la présence d'objets de tailles hydrodynamiques 204 nm pour 5% d'entre eux et de 1810 nm pour 95% d'entre eux. Le point isoélectrique est estimé à pH 2,5 et le potentiel zéta diminue de 5 mV à pH 2 à -30 mV à pH 12. Les mesures au CHNS révèlent un pourcentage de carbone de 3,3%. Lorsqu'une suspension contenant les parties centrales de magnétosomes revêtues d'Al(OH)₃ est mélangée à des cellules GL-261 et soumise à la condition 3 de traitement, la température du mélange augmente de 21,3 °C après 30 minutes d'application du champ. La pente à l'origine de la variation de température est estimée à 0,034 °C/sec. Lorsque ce même mélange est soumis à la condition 2 de traitement, le pourcentage de cellules vivantes est de 26% alors qu'il est de 91% sans application du champ (condition 1 de traitement). Cela indique la cytotoxicité induite par les parties centrales des magnétosomes revêtus d'Al(OH)₃ sur les cellules GL261 en présence de la condition 2 de traitement.

### Exemple 20: Parties centrales des magnétosomes issues de MSR-1, revêtues de silice (APTS)

**Préparation:** La suspension contenant 7 mg par millilitre de la partie centrale des magnétosomes est d'abord soniquée pendant 5 minutes à une puissance de 5 W avec des pulses de 0,1 seconde et des intervalles entre pulses de 0,1 seconde. Dans un tube en verre de 8 mL, une suspension contenant 10 mg de la partie centrale des magnétosomes est introduite, la suspension est placée contre un aimant au néodyme, le surnageant est retiré et remplacé par 2 mL d'un mélange d'hexane et d'éthanol absolu (1/1 ; v/v). La suspension est soniquée pendant quelques minutes avant ajout de 200 µL d'APTS (3-Aminopropyl)triethoxysilane, APTS ; référence Sigma: 440140) soit 211 mg et 500µL de NaOH à 5M. Le mélange est soniqué pendant 10 minutes à 85°C et à une puissance de 5 W avec des pulses de 0,1 seconde et des intervalles entre pulses de 0,1 seconde. On effectue un premier lavage en plaçant la suspension contre un aimant, en retirant le surnageant et en le remplaçant par un volume de 1,5 mL d'un mélange d'hexane et d'éthanol absolu (1/1 ; v/v) puis la suspension est soniquée pendant 30 secondes à une puissance de 5 W avec des pulses de 0,1 seconde et des intervalles entre pulses de 0,1 seconde. On effectue trois lavages supplémentaires de la même manière. Ensuite, un volume de 1 millilitre d'hydroxyde de sodium à 5M est ajouté à la suspension de nanoparticules puis le mélange est soniqué pendant 15 minutes à 80°C à une puissance de 5W avec des pulses de 0,1 seconde et des intervalles entre pulses de 0,1 seconde. On effectue un premier lavage en plaçant la suspension contre un aimant, en retirant le surnageant et en le remplaçant par de l'eau hyclone. On effectue deux lavages supplémentaires de la même manière.

**Caractérisation:** L'absorption de la suspension des parties centrales des magnétosomes revêtues de silice contenant 1 mg der fer, mesurée à 480 nm, diminue de 90% pendant les 20 minutes de mesure. Les mesures de microscopie électronique révèlent la présence d'un gel entourant les parties centrales de ces magnétosomes. Les mesures de diffusion de lumière de cette suspension indiquent la présence d'objets de tailles hydrodynamiques 235 nm pour 10% d'entre eux, de 277 nm pour 7% d'entre eux et de 1986 nm pour 93% d'entre eux. Le point isoélectrique est estimé à pH 6,7 et le potentiel zéta diminue de 39 mV à pH 2 à -31 mV à pH 12. Les mesures au CHNS révèlent un pourcentage de carbone de 7,4%. Lorsqu'une suspension contenant les parties centrales de magnétosomes revêtues de silice est mélangée à des cellules GL-261 et soumise à la condition 3 de traitement, la température du mélange augmente de 26,9 °C après 30 minutes d'application du champ. La pente à l'origine de la variation de température est estimée à 0,1 °C/sec. Lorsque ce même mélange est soumis à la condition 2 de traitement, le pourcentage de cellules vivantes est de 2,5% alors qu'il est de 40% sans application du champ (condition 1 de traitement). Cela indique la cytotoxicité induite par les parties centrales des magnétosomes revêtus de silice sur les cellules GL261 en présence de la condition 2 de traitement.

### Exemple 21 : Différents protocoles de revêtement.

A partir des protocoles décrits dans les exemples 6 à 20, il peut être possible de revêtir la partie centrale des agents de revêtement poly-L-lysine, chitosan, carboxy-methyl-dextran, acide citrique, acide oléique, silice, acide folique, DOPC, alendronate, neridronate, PEI, Al(OH)₃ en utilisant pour le mélange de la partie centrale et de l'agent de revêtement un rapport entre masse de revêtement et masse de partie centrale compris entre 10⁻⁹ et 10⁹, entre 10⁻⁶ et 10⁶, ou entre 10⁻² et 10², un temps de sonication compris entre 10⁻⁹ et 10⁹ secondes, entre 10⁻⁶ et 10⁶ secondes, ou entre 10⁻² et 10² secondes ou une puissance de sonication comprise entre 10⁻⁹ et 10⁹ W, entre 10⁻⁶ et 10⁶ W, ou entre 10⁻² et 10² W.

### Exemple 22 : Propriétés de liaison des parties centrales entre elles.

Des mesures MET effectuées sur des parties centrales revêtues de différents revêtements (PEI, DOPC, Néridronate, Chitosan, acide citrique, dextran, AlOH₃, Silice, acide folique) révèlent que la distance séparant la surface externe de deux parties centrales séparées par du matériau de liaison est comprise entre 0 et plus de 400 nm, que le nombre de parties centrales liées entre elles par du matériau de liaison est compris entre 2 et plus de 10 000 et que les parties centrales liées entre elles par du matériau de liaison forment différentes figures géométriques telles que des chaînes, cercles, losanges, quadrilatères (tableau 3), que les chaînes sont caractérisées par la présence de différentes parties centrales dont les facettes sont parallèles, ce qui suggère un alignement des axes cristallographiques des différentes parties centrales dans la direction d'élongation des chaînes.

**Tableau 1**

| Propriétés des échantillons | | | Pyrogénicité (test LAL) | Stabilité (Δabs à 480 nm) | Propriétés de chauffage et efficacité anti-tumorale (*in vitro*) | | | |
|---|---|---|---|---|---|---|---|---|
| Type d'échantillon | Revêtement | Espèce | Niveau d'endotoxines (UE/mg/mL) | Δabs (%) | ΔT (°C) | δT/δt (°C.s⁻¹) | %cellules vivantes(-B) | %cellules vivantes(+B) |
| BNF-Starch | hydroxyéthyl amidon | chimique | <50 | 0 | 6,2 | 0,009 | 71 ± 5 | 78 ± 5 |
| *BNF-Starch* | *hydroxyéthyl amidon* | *chimique* | | *0* | *7,6* | *0,01* | *86* | *31* |
| Bactéries entières | | AMB-1 | | | | | | |
| Bactéries entières | | MSR-1 | | | | | | |
| Chaînes pyrogènes extraites | Membrane bactérienne | AMB-1 | 18000-150000 | 30 | 20,5 | 0,043 | | |
| *Chaînes pyrogènes extraites* | *Membrane bactérienne* | *AMB-1* | | | *9,8* | *0,012* | *55* | *10* |
| Chaînes pyrogènes extraites | Membrane bactérienne | MSR-1 | 2000-11300 | | 9,4 | 0,019 | 39 ± 5 | 5 ± 5 |
| *Chaînes pyrogènes extraites* | *Membrane bactérienne* | *MSR-1* | *2000 - 17000* | *86* | | *0,012* | *39* | *12* |
| Parties centrale des magnétosomes | Aucun | AMB-1 | | 80-90 | | | | |
| *Parties centrale des magnétosomes* | *Aucun* | *AMB-1* | *20 à 100* | *66-93* | *23,8* | *0,024* | *30* | *48* |
| Parties centrales des magnétosomes | Aucun | MSR-1 | 10 à 100 | 60-80 | | | | |
| *Parties centrales des magnétosomes* | *Aucun* | *MSR-1* | | | *9,8* | *0,012* | *77* | *0 à 9* |
| Parties centrales des magnétosomes | Poly-L-lysine | MSR-1 | 78 | 50 | 11 | 0,024 | 53 ± 5 | 23 ± 5 |
| *Parties centrales des magnétosomes* | *Poly-L-lysine* | *MSR-1* | | *30* | | | | |
| Parties centrales des magnétosomes | Chitosan | MSR-1 | | 3 | 8,5 | 0,009 | 20 ± 5 | 0 à 5 |
| *Parties centrales des magnétosomes* | *Chitosan* | *MSR-1* | 25 | | | *0,014* | | |
| Parties centrales des magnétosomes | Carboxy-méthyldextran | MSR-1 | | 0 | 28,8 | 0,023 | 63 ± 5 | 11 ± 5 |
| *Parties centrales des magnétosomes* | *Carboxy-méthyldextran* | *MSR-1* | | *10* | | | | |
| Parties centrales des magnétosomes | Acide citrique | MSR-1 | 19 | 15 | 25,2 | 0,038 | 57 ± 5 | 26 ± 5 |
| *Parties centrales des magnétosomes* | *Acide citrique* | *MSR-1* | | | | | *57* | *7* |
| Parties centrales des magnétosomes | Acide oléique | MSR-1 | | 0 | 28,4 | 0,051 | | |
| *Parties centrales des magnétosomes* | *Acide oléique* | *MSR-1* | | *0* | *5* | *0.012* | *53* | *14* |
| Parties centrales des magnétosomes | Silice | MSR-1 | | | | | | |
| *Parties centrales des magnétosomes* | *Silice* | *MSR-1* | | *90* | *26,9* | *0,100* | *40* | *2,5* |
| Parties centrales des magnétosomes | Acide folique | MSR-1 | | 0 | 20 | 0,042 | 93 ± 5 | 9 ± 5 |
| Parties centrales des magnétosomes | DOPC | MSR-1 | | 0 | 33,5 | 0,049 | 13 ± 5 | 0 à 5 |
| *Parties centrales des magnétosomes* | *DOPC* | *MSR-1* | | *2-70* | | | | |
| Parties centrales des magnétosomes | DOPC | AMB-1 | | | | | | |
| Parties centrales des magnétosomes | Alendronate | AMB-1 | 53 - 144 | | | | | |
| *Parties centrales des magnétosomes* | *Alendronate* | *AMB-1* | | *1* | *18,3* | *0,028* | *17* | *2* |
| Parties centrales des magnétosomes | Néridronate | MSR-1 | | 0 | 6,7 | 0,017 | 44 ± 5 | 28 ± 5 |
| Parties centrales des magnétosomes | PEI | MSR-1 | | 0 | 6 | 0,014 | 40 ± 5 | 19 ± 5 |
| Parties centrales des magnétosomes | PEI | AMB-1 | <50 | | | | | |
| *Parties centrales des magnétosomes* | *PEI* | *AMB-1* | | *64* | *12* | *0,040* | *26* | *12* |
| Parties centrales des magnétosomes | Al(OH)₃ | MSR-1 | | 0 | | | | |
| *Parties centrales des magnétosomes* | *Al(OH)₃* | *MSR-1* | | | *21,3* | *0,034* | *91* | *26* |

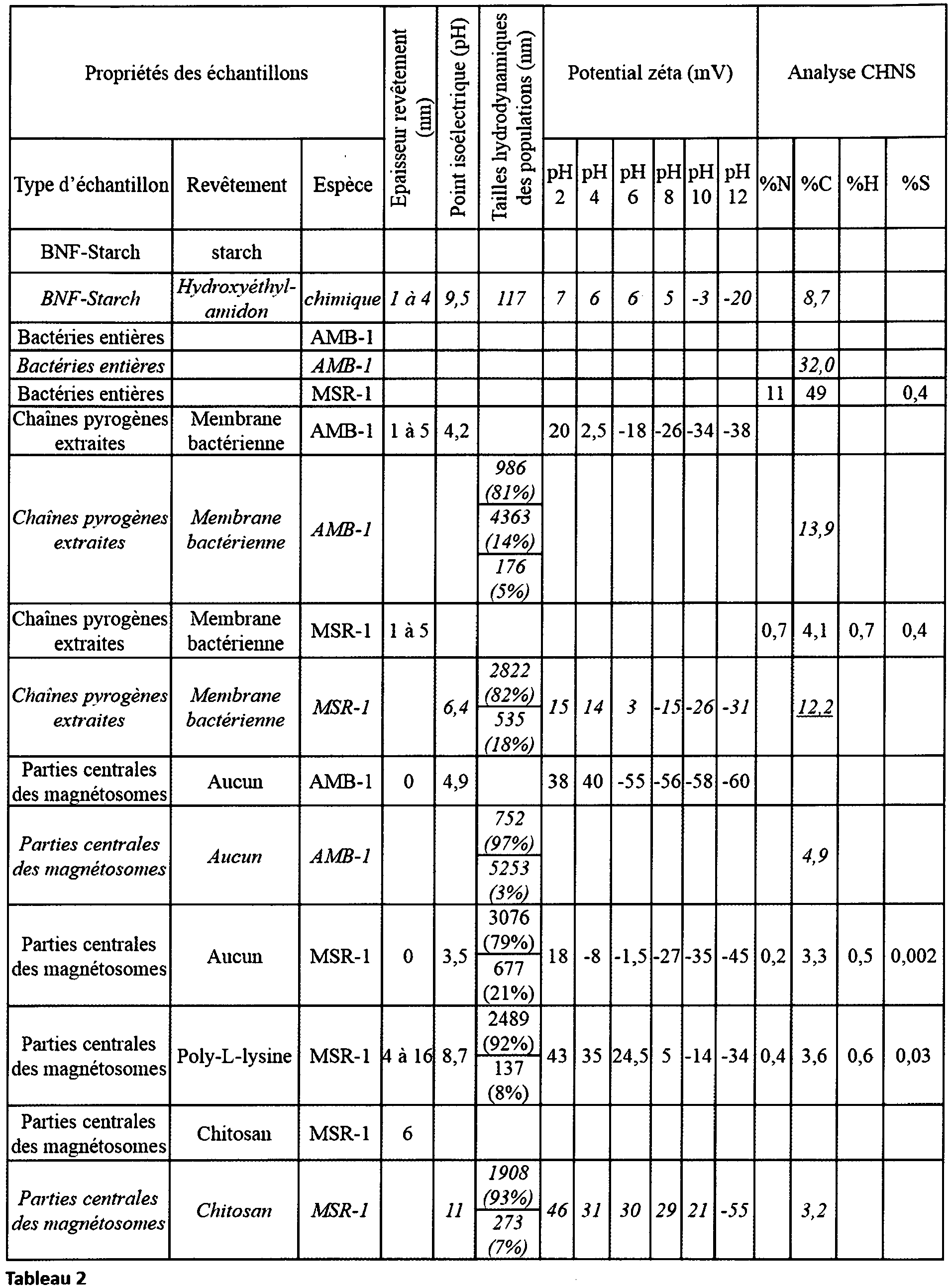

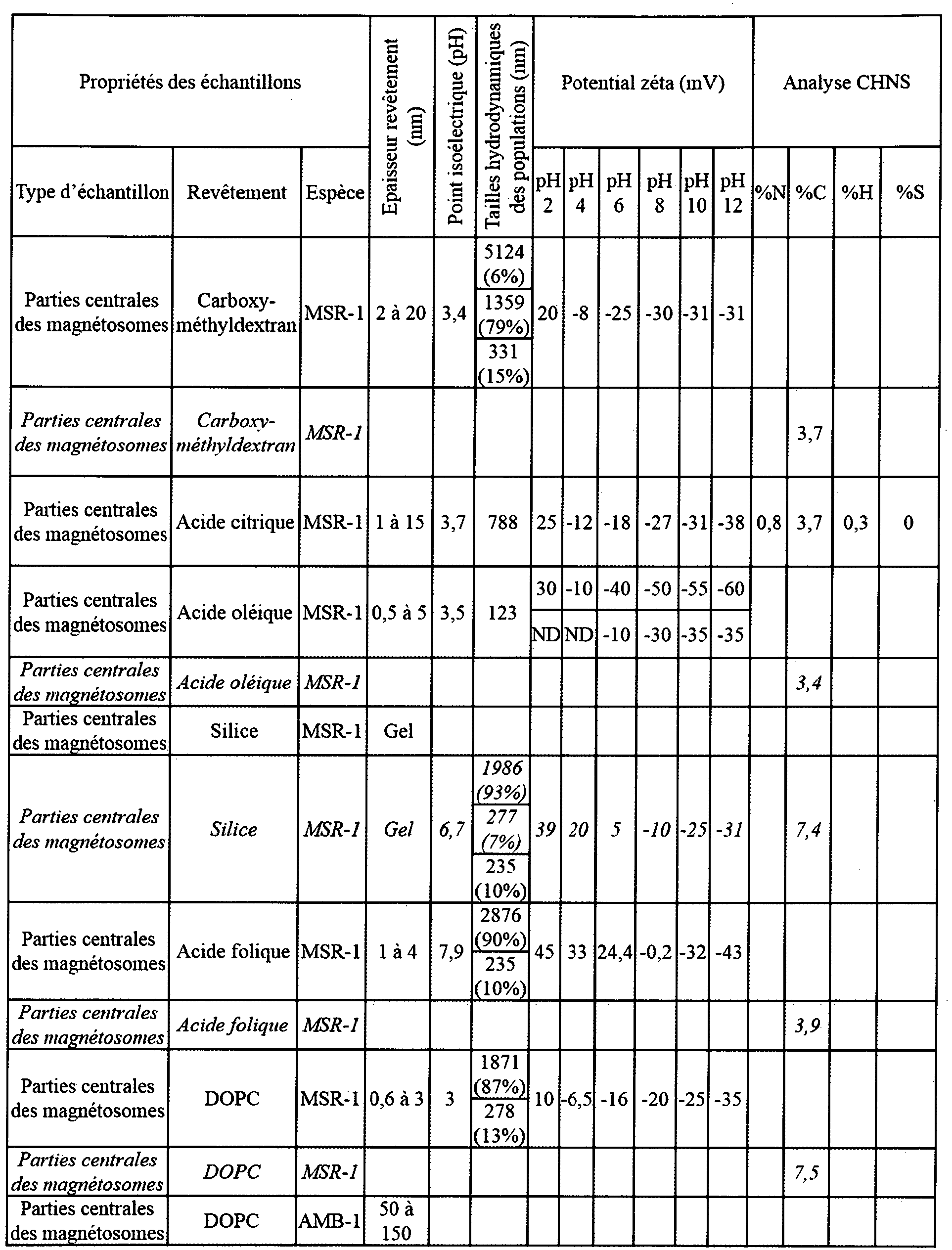

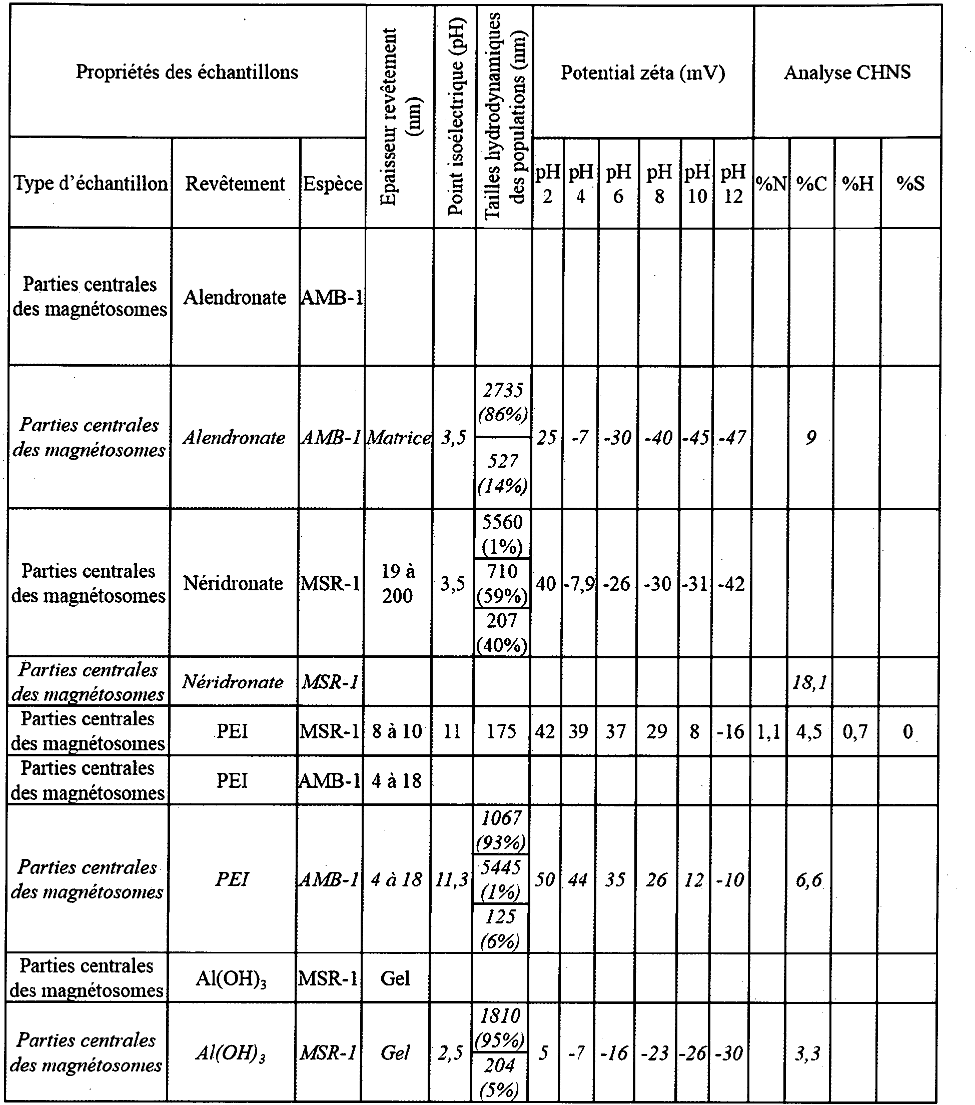

**Tableau 3**

| Revêtement | Espèce bactérienne | Distance séparant la surface externe de deux parties centrales séparées par du matériau de liaison | | Nombre de parties centrales liées entre elles par du matériau de liaison | | Formes géométriques |
|---|---|---|---|---|---|---|
| | | Min | Max | Min | Max | |
| **PEI** | MSR1 | 0 | 16 | 2 | 26 | Chaînes individuelles, chaînes collées l'une à l'autre, chaînes, agrégats sphériques, cercles |
| **PEI** | AMB1 | 0 | 191 | 2 | 5 | Chaînes, cercles |
| **DOPC** | MSR1 | 0 | 27 | 2 | 16 | Cercle, chaîne, losange |
| **Néridronat e** | MSR1 | 0 | 31 | 2 | 18 | Chaînes, chaînes collées l'une à l'autre |
| **Chitosan** | MSR1 | 0 | 11 | 2 | 42 | Chaînes, chaînes collées l'une à l'autre |
| **Acide citrique** | MSR1 | 0 | 16 | 2 | 26 | Chaînes, cercles, triangle, quadrilatère |
| **Dextran** | MSR1 | 0 | 113 | 2 | 8 | Cercles, chaînes |
| **AlOH₃** | MSR1 | 0 | >200 nm | NA | >10000 | Chaîne, cercle dans un gel |
| **Silice** | MSR1 | 0 | >50 nm | NA | >10000 | Chaîne, cercle dans un gel |
| **Acide folique** | MSR1 | 0 | >400 nm | NA | >10000 | Chaîne, cercle dans un gel |

## Revendications

1. Préparation comprenant au moins une nanoparticule synthétique, dans laquelle la nanoparticule comprend :
- une partie centrale minérale cristallisée comprenant majoritairement un oxyde de fer, synthétisée par un organisme vivant, et
- un revêtement périphérique soit comprenant moins de 10% de matière organique non dénaturée provenant de l'organisme vivant soit ne comprend pas de protéines provenant de l'organisme vivant,
dans lequel ledit organisme vivant est une bactérie magnétotactique.

2. Préparation selon la revendication **1** dans laquelle le revêtement périphérique comprend moins de 1% de protéines non dénaturés provenant des bactéries magnétotactiques.

3. Préparation selon la revendication **1** ou **2,** dans laquelle la partie centrale comprend de la maghémite et/ou de la magnétite.

4. Préparation selon l'une des revendications **1** à **3,** dans laquelle la nanoparticule synthétique est apyrogène.

5. Préparation selon la revendication **4,** dans laquelle la nanoparticule synthétique présente une concentration en endotoxines inférieure à 10⁹ unités d'endotoxines par milligramme d'oxyde de fer ou unités d'endotoxines par millilitre ou unités d'endotoxines par milligramme d'oxyde de fer par millilitre.

6. Préparation selon l'une des revendications **1** à **5,** dans laquelle les nanoparticules synthétiques sont arrangées suivant une figure géométrique sélectionnée parmi une chaîne droite, un cercle, un carré, un rectangle, un triangle, un pentagone, un hexagone, un heptagone, un octogone, un polygone ou un polyèdre.

7. Préparation selon l'une des revendications **1** à **6,** dans laquelle le revêtement comprend au moins un composé capable d'établir des interactions faibles ou des liaisons covalentes avec la partie centrale des nanoparticules synthétiques.

8. Préparation selon l'une des revendications **1** à **7,** dans laquelle le revêtement comprend au moins un composé capable d'établir des interactions ou des liaisons avec les ions Fe2+ ou Fe³⁺, hydroxyles OH⁻, oxydes O²⁻, des défauts cristallins de la partie centrale.

9. Préparation selon l'une des revendications **1** à **8,** dans laquelle le revêtement comprend des composés carbonés.

10. Préparation selon l'une des revendications **1** à **9,** dans laquelle le revêtement comprend au moins un composé sélectionné dans le groupe constitué d'un chélatant, d'une molécule amphiphatique, d'un polymère polarisé ou chargé, d'un oxyde de métal ou de silicium, d'un hydroxyde de métal ou de silicium, d'un acide, d'un dérivé acide, basique, oxydé, réduit, neutre, chargé positivement, chargé négativement de ces composés et d'une combinaison de plusieurs de ces composés ou de ses dérivés.

11. Préparation selon l'une des revendications **1** à **10,** dans laquelle le revêtement comprend au moins un composé sélectionné dans le groupe constitué d'un polysaccharide, d'un acide gras, d'un phospholipide, d'un polymère d'acides aminés, de silice polymérique ou non et d'un polymère aliphatique aminé, d'un dérivé acide, basique, oxydé, réduit, neutre, chargé positivement, chargé négativement de ces composés et d'une combinaison de plusieurs de ces composés ou de ses dérivés.

12. Préparation selon au moins l'une des revendications **1** à **11,** dans laquelle le revêtement comprend au moins une fonction sélectionné dans le groupe constitué des fonctions acide carboxylique, phosphorique, sulfonique esters, amides, cétones, alcools, phénols, thiols, amines, éthers, sulfures, anhydrides d'acides, halogénures d'acyles, amidines, nitriles, hydropéroxydes, imines, aldéhydes, peroxydes, d'un dérivé acide, basique, oxydé, réduit, neutre, chargé positivement, chargé négativement de ces composés, et d'une combinaison de plusieurs de ces composés ou de ses dérivés.

13. Préparation selon l'une des revendications **1** à **12,** pour son utilisation à titre de médicament ou d'agent diagnostic, notamment dans le cadre du traitement d'une tumeur, par exemple par hyperthermie magnétique.

14. Utilisation de la préparation selon l'une des revendications **1** à **13** pour des applications cosmétiques.

15. Composition pharmaceutique ou médicament comprenant, à titre de principe actif, une préparation telle que définie dans l'une des revendications **1** à **13** et éventuellement au moins un véhicule pharmaceutiquement acceptable.

16. Dispositif médical comprenant une préparation telle que définie dans l'une des revendications **1** à **13.**

17. Composition de diagnostic comprenant une préparation telle que définie dans l'une des revendications **1** à **13.**

18. Composition cosmétique comprenant à titre de principe actif cosmétique une préparation telle que définie dans l'une des revendications **1** à **13.**

19. Procédé de fabrication d'une préparation telle que définie dans l'une des revendications **1** à **13,** comprenant les séquences suivantes :
(i), à partir d'une préparation de nanoparticules synthétisées par un organisme vivant comprenant une partie centrale minérale cristallisée composée majoritairement d'un oxyde de fer et un revêtement périphérique biologique, isoler la partie centrale minérale ;
(ii), traiter la préparation obtenue de manière à recouvrir la partie centrale par un revêtement périphérique ;
(iii), éventuellement stériliser la préparation, préférentiellement après la séquence (i), possiblement après la séquence (ii).

## Patentansprüche

1. Präparat, mindestens ein synthetisches Nanopartikel enthaltend, in dem das Nanopartikel umfasst:
- einen mineralischen, kristallinen Zentralteil, der überwiegend ein Eisenoxid umfasst und der von einem lebenden Organismus synthetisiert wurde, und
- eine periphere Beschichtung, die entweder mindestens 10% nicht denaturierter, aus dem lebenden Organismus stammender organischer Materie enthält, oder keine aus dem lebenden Organismus stammende Proteine,
wobei der lebende Organismus eine magnetotaktische Bakterie ist.

2. Präparat nach Patentanspruch 1, in dem die periphere Beschichtung weniger als 1% nicht denaturierter, aus den magnetotaktischen Bakterien stammender Proteine enthält.

3. Präparat nach Patentanspruch 1 oder 2, in dem der Zentralteil Maghämit und/oder Magnetit enthält.

4. Präparat nach einem der Patentansprüche 1 bis 3, in dem das synthetische Nanopartikel apyrogen ist.

5. Präparat nach Patentanspruch 4, in dem das synthetische Nanopartikel eine Konzentration an Endotoxinen kleiner als 10⁹ Endotoxineinheiten pro Milligramm Eisenoxid oder Endotoxineinheiten pro Milliliter oder Endotoxineinheiten pro Milligramm Eisenoxid pro Milliliter aufweist.

6. Präparat nach einem der Patentansprüche 1 bis 5, in dem die synthetischen Nanopartikel in einer geometrischen Figur angeordnet sind, ausgewählt unter gerader Kette, Kreis, Quadrat, Rechteck, Dreieck, Fünfeck, Sechseck, Siebeneck, Achteck, Polygon oder Polyeder.

7. Präparat nach einem der Patentansprüche 1 bis 6, in dem die Beschichtung mindestens eine Verbindung enthält, die fähig ist, schwache Wechselwirkungen oder Kovalentbindungen mit dem Zentralteil der synthetischen Nanopartikel einzugehen.

8. Präparat nach einem der Patentansprüche 1 bis 7, in dem die Beschichtung mindestens eine Verbindung enthält, die fähig ist, schwache Wechselwirkungen oder Bindungen mit den Ionen Fe²⁺ oder Fe³⁺, Hydroxylionen OH⁻, Oxydionen O²⁻, Kristallfehlern des Zentralteils einzugehen.

9. Präparat nach einem der Patentansprüche 1 bis 8, in dem die Beschichtung Kohlenstoffverbindungen enthält.

10. Präparat nach einem der Patentansprüche 1 bis 9, in dem die Beschichtung mindestens eine Verbindung enthält, gewählt aus der Gruppe, bestehend aus Chelator, amphipathischem Molekül, polarisiertem oder geladenem Polymer, Metall- oder Siiiziumoxid, Metall- oder Siiiziumhydroxid, Säure, saurem, basischem, oxydiertem, reduziertem, neutralem, positiv geladenem oder negativ geladenem Derivat dieser Verbindungen und einer Kombination mehrerer dieser Verbindungen und Derivate.

11. Präparat nach einem der Patentansprüche 1 bis 10, in dem die Beschichtung mindestens eine Verbindung enthält, gewählt aus der Gruppe, bestehend aus Polysaccharid, Fettsäure, Phospholipid, Polymer aus Aminosäuren, polymerem oder nicht-polymerem Silizium und aliphatischem Aminopolymer, saurem, basischem, oxydiertem, reduziertem, neutralem, positiv geladenem oder negativ geladenem Derivat dieser Verbindungen und einer Kombination mehrerer dieser Verbindungen und Derivate.

12. Präparat nach mindestens einem der Patentansprüche 1 bis 11, in dem die Beschichtung mindestens eine funktionelle Gruppe enthält, gewählt aus der Gruppe, bestehend aus Karboxyl-, Phosphorsäuregruppe, Sulfonsäureester, Amid-, Keto-, Alkohol-, Phenol-, Thiol-, Amino-, Ether-, Schwefel-, Säureanhydrid-, Akylhalogenid-, Amidin-, Nitril-, Hydroperoxyd-, Imin-, Aldehyd, Peroxydgruppen, saurem, basischem, oxydiertem, reduziertem, neutralem, positiv geladenem oder negativ geladenem Derivat dieser Verbindungen und einer Kombination mehrerer dieser Verbindungen und Derivate.

13. Präparat nach einem der Patentansprüche 1 bis 12 zum Gebrauch als Medikament oder Diagnosereagenz, insbesondere im Rahmen der Behandlung eines Tumors, beispielsweise durch magnetische Hyperthermie.

14. Gebrauch des Präparates nach einem der Patentansprüche 1 bis 13 für Kosmetikanwendungen.

15. Pharmazeutische Zubereitung oder Medikament, als Wirkstoff ein Präparat nach einem der Patentansprüche 1 bis 13 enthaltend und gegebenenfalls mindestens ein pharmazeutisch verträgliches Vehikel.

16. Medizinische Vorrichtung, ein Präparat nach einem der Patentansprüche 1 bis 13 enthaltend.

17. Diagnosezubereitung, ein Präparat nach einem der Patentansprüche 1 bis 13 enthaltend.

18. Kosmetische Zubereitung, als kosmetischen Wirkstoff ein Präparat nach einem der Patentansprüche 1 bis 13 enthaltend.

19. Verfahren zur Herstellung eines Präparates nach einem der Patentansprüche 1 bis 13, folgende Schritte umfassend:
(i) Isolation aus einem Präparat von einem lebenden Organismus synthetisierter Nanopartikel, die einen mineralischen, kristallinen Zentralteil, überwiegend aus einem Eisenoxid bestehend, und eine periphere, biologische Beschichtung umfassen, des mineralischen Zentralteils,
(ii) Behandeln des gewonnenen Präparates, um den Zentralteil mit einer peripheren Beschichtung zu bedecken,
(iii) gegebenenfalls Sterilisation des Präparates, vorzugsweise nach Schritt (i), möglicherweise nach Schritt (ii).

## Claims

1. Preparation comprising at least one synthetic nanoparticle, in which the nanoparticle comprises:
- a crystallized mineral central part mainly comprising an iron oxide, synthetized by a living organism, and
- a peripheral coating either comprising less than 10% of non-denatured organic material originating from the living organism or not comprising proteins originating from the living organism,
wherein said living organism is a magnetotactic bacterium.

2. Preparation according to claim **1,** wherein the peripheral coating comprises less than 1% of non-denatured protein originating from magnetotactic bacteria.

3. Preparation according to claim **1** or **2,** in which the central part comprises maghemite and / or magnetite.

4. Preparation according to any one of claims **1** to **3,** wherein the synthetic nanoparticle is apyrogenic.

5. Preparation according to claim **4,** wherein the synthetic nanoparticle has an endotoxin concentration lower than 10⁹ endotoxin units per milligram of iron oxide or endotoxin units per milliliter or endotoxin units per milligram of iron oxide per milliliter.

6. Preparation according to any one of claims **1** to **5,** in which the synthetic nanoparticles are arranged according to a geometric figure selected from a straight chain, a circle, a square, a rectangle, a triangle, a pentagon, a hexagon, a heptagon, an octagon, a polygon or a polyhedron.

7. Preparation according to any one of claims **1** to **6,** wherein the coating comprises at least one compound capable of establishing weak interactions or covalent bonds with the central part of the synthetic nanoparticles.

8. Preparation according to any one of claims **1** to **7,** wherein the coating comprises at least one compound capable of establishing interactions or bonds with Fe²⁺ or Fe³⁺ ions, OH⁻hydroxyls, O²⁻ oxides, crystalline defects of the central part.

9. Preparation according to any one of claims **1** to **8,** wherein the coating comprises carbonaceous compounds.

10. Preparation according to any one of claims **1** to **9,** wherein the coating comprises at least one compound selected from the group consisting of a chelating agent, an amphiphatic molecule, a polarized or charged polymer, a metal oxide or silicon, a metal or silicon hydroxide, an acid, an acidic, basic, oxidized, reduced, neutral, positively charged, negatively charged derivative of these compounds and a combination of several of these compounds or their derivatives.

11. Preparation according to any one of claims **1** to **10,** wherein the coating comprises at least one compound selected from the group consisting of a polysaccharide, a fatty acid, a phospholipid, a polymer of amino acids, of polymeric or non-polymeric silica and of an amino aliphatic polymer, of an acid, basic, oxidized, reduced, neutral, positively charged or negatively charged derivative of these compounds and of a combination of several of these compounds or of their derivatives.

12. Preparation according to any one of claims **1** to **11,** in which the coating comprises at least one function selected from the group consisting of carboxylic acid, phosphoric acid, sulfonic acid functions, amides, ketones, alcohols, phenols, thiols, amines, ethers , sulfides, acid anhydrides, acyl halides, amidines, nitriles, hydroperoxides, imines, aldehydes, peroxides, of an acidic, basic, oxidized, reduced, neutral, positively charged, negatively charged derivative of these compounds, and a combination of several of these compounds or their derivatives.

13. Preparation according to any one of claims **1** to **12,** for its use as a medicament or diagnostic agent, in particular in the context of the treatment of a tumor, for example with magnetic hyperthermia.

14. Use of the preparation according to any one of claims **1** to **13** for cosmetic applications.

15. Pharmaceutical composition or medicament comprising, as active principle, a preparation as defined in any one of claims **1** to **13** and optionally at least one pharmaceutically acceptable vehicle.

16. A medical device comprising the preparation as defined in any one of claims **1** to **13.**

17. A diagnostic composition comprising the preparation as defined in any one of claims **1** to **13.**

18. Cosmetic composition comprising, as cosmetic active principle, the preparation as defined in any one of claims **1** to **13.**

19. Process for manufacturing the preparation as defined in any one of claims **1** to **13,** comprising the following sequences:
(i), from a preparation of nanoparticles synthesized by a living organism comprising a crystallized mineral central part composed mainly of an iron oxide and a biological peripheral coating, isolating the mineral central part;
(ii) treating the obtained preparation so as to cover the central part with a peripheral coating;
(iii), optionally sterilize the preparation, preferably after sequence (i), possibly after sequence (ii).
